# FASCICULE DE BREVET EUROPEEN

(11) **EP 1 466 604 B1**
(45) Date de publication et mention de la délivrance du brevet: **07.11.2007**
(21) Numéro de dépôt: 04290918.4
(22) Date de dépôt: 07.04.2004
(51) Int. Cl.: A61K 31/472, C07D 217/16, A61P 25/00, A61P 3/04, A61P 35/00

(54) **Nouveaux dérivés d'isoquinoléine, leur procédé de préparation et leur utilisation pour traiter les troubles du système mélatoninergique**
Isochinolinderivate, Verfahren zu ihrer Herstellung und ihre Verwendung um Melatonin System-Probleme zu behandeln
Isoquinoline derivatives, process for their preparation and use thereof in the treatment of melatoninergic system problems

(30) Priorité: 09.04.2003 FR 0304381
(43) Date de publication de la demande: 13.10.2004
(73) Titulaire: Les Laboratoires Servier, 92415 Courbevoie Cedex (FR)
(72) Inventeur: Poissonnier-Durieux, Sophie, 80260 Rainneville (FR); Wallez, Valérie, 59117 Wervicq-Sud (FR); Gasnereau, Anne, 59000 Lille (FR); Yous, Said, 59120 Loos (FR); Lesieur, Daniel, 59147 Gondecourt (FR); Delagrange, Philippe, 92130 Issy les Moulineaux (FR); Renard, Pierre, 78150 Le Chesnay (FR); Bennejean, Caroline, 94220 Charenton le Pont (FR); Boutin, Jean Albert, 92150 Suresnes (FR); Audinot, Valérie, 78300 Poissy (FR)

(56) Documents cités:
- EP-A- 0 447 285
- EP-A- 0 721 938
- WO-A-97/01539
- WO-A-99/58495

## Description

La présente invention concerne de nouveaux dérivés d'isoquinoléine, leur procédé de préparation et les compositions pharmaceutiques qui les contiennent.

On connaît dans la littérature des dérivés d'isoquinoléine utiles en tant que vasodilateurs (US 4,880,817, US 4,843,071, US 4,822,800), ou utiles dans la croissance des plantes (Czasopismo Techniezne (Krakow), 1992, 89 (1), 7-12), en tant que modulateurs de tyrosine phosphatase (WO 9946268), ou encore utiles en synthèse (Tetrahedron Letters, 2002, 43 (19), 3557-3560 ; Heterocycles, 2000, 52 (3), 1371-1383).

Les composés de la présente invention, de par leur structure originale, sont nouveaux et présentent des propriétés pharmacologiques très intéressantes concernant les récepteurs mélatoninergiques.

De nombreuses études ont mis en évidence ces dix dernières années le rôle capital de la mélatonine (N-acétyl-5-méthoxytryptamine) dans de nombreux phénomènes physiopathologiques ainsi que dans le contrôle du rythme circadien. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.

Outre leur action bénéfique sur les troubles du rythme circadien (J. Neurosurg. 1985, 63, pp. 321-341) et du sommeil (Psychopharmacology, 1990, 100, pp. 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp. 264-272), et analgésiques (Pharmacopsychiat., 1987, 20, pp. 222-223) ainsi que pour le traitement des maladies de Parkinson (J. Neurosurg. 1985, 63, pp. 321-341) et d'Alzheimer (Brain Research, 1990, 528, pp. 170-174). De même, ces composés ont montré une activité sur certains cancers (Melatonin - Clinical Perspectives, Oxford University Press, 1988, pp. 164-165), sur l'ovulation (Science 1987, 227, pp. 714-720), sur le diabète (Clinical Endocrinology, 1986, 24, pp. 359-364), et dans le traitement de l'obésité (International Journal of Eating Disorders, 1996, 20 (4), pp. 443-446).
Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol. Sci., 1995, 16, p. 50 ; WO 97.04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands sélectifs. De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Des composés utiles pour le traitement de troubles liés au système mélatominergique ont été décrits dans l'art antérieur (WO 99/58495).

Les composés de la présente invention outre leur nouveauté, montrent une très forte affinité pour les récepteurs de la mélatonine et/ou une sélectivité pour l'un ou l'autre des sous-types réceptoriels mélatoninergiques.

Plus particulièrement, la présente invention concerne les composés de formule (I) : 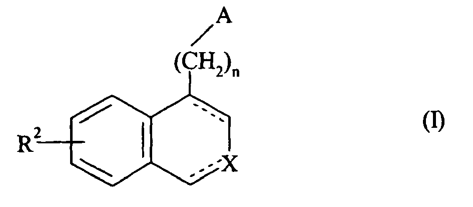 dans laquelle :
◆ n vaut 1, 2 ou 3,
◆ A représente un groupement 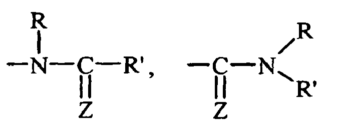 ou un groupement 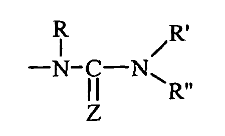 dans lesquels :
   - Z représente un atome de soufre ou d'oxygène,
   - R et R", identiques ou différents, représentent chacun un atome d'hydrogène ou un groupement alkyle (C₁-C₆) linéaire ou ramifié,
   - et R' représente un groupement alkyle (C₁-C₆) linéaire ou ramifié, alkényle (C₂-C₆) linéaire ou ramifié, alkynyle (C₂-C₆) linéaire ou ramifié, cycloalkyle (C₃-C₈), cycloalkyl(C₃-C₈)alkyle(C₁-C₆) dont la partie alkyle est linéaire ou ramifiée, aryle, arylalkyle (C₁-C₆) dont la partie alkyle est linéaire ou ramifiée, hétéroaryle ou hétéroarylalkyle (C₁-C₆) dont la partie alkyle est linéaire ou ramifiée,
◆ X représente un atome d'azote ou un groupement N-R¹ dans lequel R¹ représente un atome d'hydrogène ou un groupement alkyle (C₁-C₆) linéaire ou ramifié, cycloalkyle (C₃-C₈), cycloalkyle (C₃-C₈) alkyle (C₁-C₆) linéaire ou ramifié, aryle, aroyle ou arylalkyle (C₁-C₆) dont la partie alkyle est linéaire ou ramifiée, hétéroaryle, hétéroaroyle ou hétéroarylalkyle (C₁-C₆) dont la partie alkyle est linéaire ou ramifiée,
◆ R² représente un groupement alkoxy (C₁-C₆) linéaire ou ramifié, cycloalkyloxy (C₃-C₈) ou cycloalkyl (C₃-C₈) alkyloxy (C₁-C₆) linéaire ou ramifié,
◆ la représentation ----- signifie que la liaison est simple ou double étant entendu que la valence des atomes est respectée,
étant entendu que :
- par "aryle", on entend un groupement phényle, ou naphtyle, ces groupements étant non substitués ou substitués par un à trois groupements identiques ou différents, choisis parmi alkyle (C₁-C₆) linéaire ou ramifié, alkoxy (C₁-C₆) linéaire ou ramifié, OH, COOH, alkoxycarbonyle dont la partie alkoxy est linéaire ou ramifiée, formyle, nitro, cyano, hydroxyméthyle, amino (éventuellement substitué par un ou deux groupements alkyle (C₁-C₆) linéaire ou ramifié), ou atomes d'halogène,
- par "hétéroaryle", on entend tout groupement mono ou bi-cyclique contenant 5 à 10 chaînons et pouvant contenir 1 à 3 hétéroatomes choisis parmi oxygène, soufre ou azote comme les groupements furane, thiophène, pyrrole, imidazoline, pyridine, quinoleine, isoquinoléine, chromane, indole, benzothiophène ou benzofurane, ces groupements pouvant être partiellement hydrogénés, non substitués ou substitués par un à trois groupements identiques ou différents, choisis parmi alkyle (C₁-C₆) linéaire ou ramifié, alkoxy (C₁-C₆) linéaire ou ramifié, OH, COOH, alkoxycarbonyle dont la partie alkoxy est linéaire ou ramifiée, formyle, nitro, cyano, amino (éventuellement substitué par un ou deux groupements alkyle (C₁-C₆) linéaire ou ramifié), hydroxyméthyle ou atomes d'halogène,
   leurs énantiomères et diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, méthanesulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les valeurs préférées de n sont 2 et 3.

Les composés préférés de l'invention sont les composés de formule (I) pour lesquels :
n vaut 2 et A représente un groupement -NHCOR' et plus particulièrement un groupement -NHCOR' dans lequel R' représente un groupement alkyle (C₁-C₆) linéaire ou ramifié comme méthyle, éthyle, propyle, isopropyle, butyle, isobutyle, pentyle ou hexyle par exemple, ou un groupement cycloalkyle (C₃-C₈) comme cyclopropyle, cyclobutyle, cyclopentyle ou cyclohexyle par exemple,
• n vaut 3 et A représente un groupement -CONHR' et plus particulièrement un groupement -CONHR' dans lequel R' représente un groupement alkyle (C₁-C₆) linéaire ou ramifié comme méthyle, éthyle, propyle, isopropyle, butyle, isobutyle, pentyle ou hexyle par exemple, ou un groupement cycloalkyle (C₃-C₈) comme cyclopropyle, cyclobutyle, cyclopentyle ou cyclohexyle par exemple.

Le groupement R² préféré est le groupement alkoxy et plus particulièrement le groupement méthoxy.

X représente préférentiellement un atome d'azote ou un groupement NR¹ dans lequel R¹ représente un groupement cycloalkylalkyle, un groupement phényle non substitué ou substitué, ou un groupement benzyle dont la partie phényle est substituée ou non substituée.

Encore plus préférentiellement, l'invention concerne les composés de formule (I) qui sont :
- le *N*-[2-(6-méthoxy-4-isoquinolinyl)éthyl]acétamide,
- le *N*-[2-(6-méthoxy-4-isoquinolinyl)éthyl]butanamide,
- le *N*-[2-(6-méthoxy-4-isoquinolinyl)éthyl]propanamide,
- le *N*-[2-(6-méthoxy-4-isoquinolinyl)éthyl]cyclopropanecarboxamide,
- le 4-(6-méthoxy-4-isoquinolinyl)-*N*-méthylbutanamide,
- le *N*-[2-(6-méthoxy-2-phényl-1,2,3,4-tétrahydro-4-isoquinolinyl)éthyl]acétamide,
- le *N*-[2-(2-benzyl-6-méthoxy-1,2,3,4-tétrahydro-4-isoquinolinyl)]éthyl]acétamide,
- le *N*-{2-[2-(cyclopropylméthyl)-6-méthoxy-1,2,3,4-tétrahydro-4-isoquinolinyl] éthyl}acétamide.

Les énantiomères, diastéréoisomères ainsi que les sels d'addition à un acide ou à une base pharmaceutiquement acceptable des composés préférés de l'invention font partie intégrante de l'invention.

La présente invention concerne également le procédé de préparation des composés de formule (I) caractérisé en ce que l'on utilise comme produit de départ le composé de formule (II) : 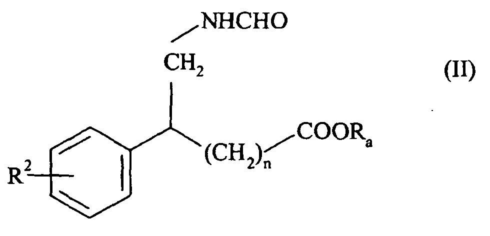 dans laquelle R² et n sont tels que définis dans la formule (I), et Rₐ représente un groupement alkyle (C₁-C₆) linéaire ou ramifié, que l'on soumet à l'action de POCl₃ pour obtenir le composé de formule (III) : 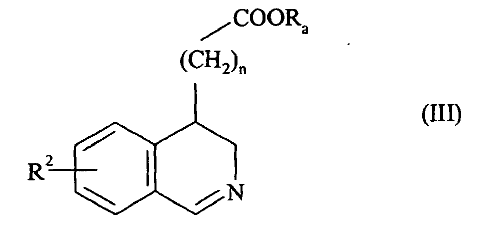 dans laquelle R², n et Rₐ sont définis comme précédemment, que l'on place :
- en présence de palladium sur charbon pour obtenir le composé de formule (IV) : 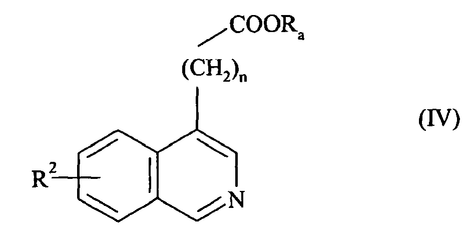
dans laquelle R², n et Rₐ sont définis comme précédemment,
- ou que l'on hydrogène en présence de palladium sur charbon pour conduire au composé et formule (V) : 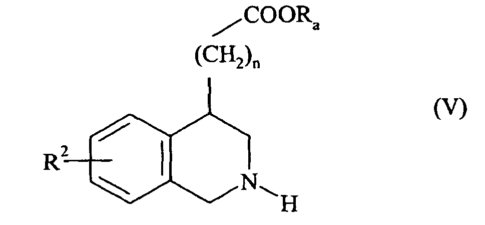
dans laquelle R², n et Rₐ sont définis comme précédemment,
composé de formule (V) sur lequel on condense un composé de formule G-R^{'1}dans laquelle G représente un groupe partant comme un atome d'halogène, ou un groupement *t*-butoxycarbonyle, et R'¹ peut prendre toutes les valeurs de R¹ à l'exception de l'atome d'hydrogène pour conduire au composé de formule (VI) : 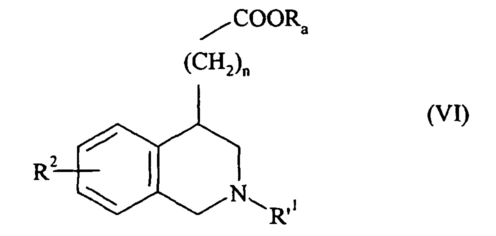 dans lequel R², R'¹, n et Rₐ sont tels que définis précédemment, les composés de formule (III) à (VI) formant le composé de formule (VII) : 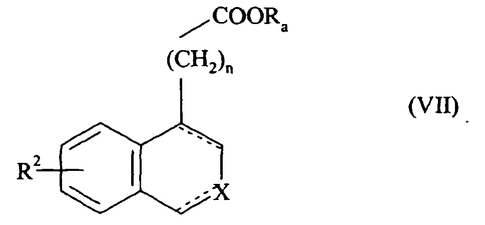 dans laquelle R², n et Rₐ sont tels que définis précédemment et X et la représentation ----- ont la même définition que dans la formule (I),
sur lequel on condense une amine de formule HNRR' dans laquelle R et R' ont la même définition que dans la formule (I) pour obtenir un composé de formule (I/a) cas particulier des composés de formule (I) : 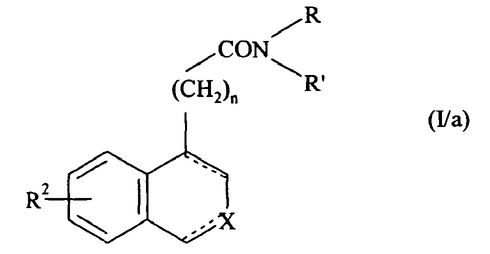 dans laquelle R², R, R', X, n et la représentation ----- sont définis comme précédemment,
ou composé de formule (VII) qui est soumis à une suite réactionnelle classique en chimie organique pour conduire au composé de formule (VIII) : 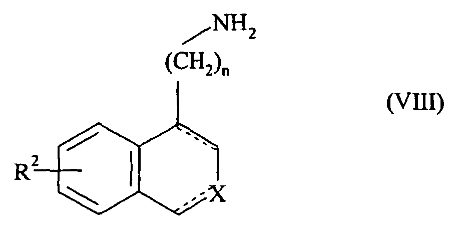 dans lequel R², X, n et la représentation ----- sont tels que définis précédemment, qui est :
- soit mis en réaction avec un chlorure d'acyle C1COR' ou l'anhydride mixte ou symétrique correspondant pour lesquels R' est tel que défini précédemment pour conduire au composé de formule (I/b), cas particulier du composé de formule (I) : 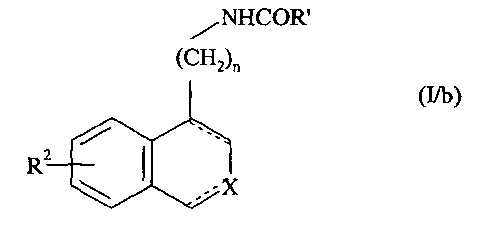 dans laquelle R², R', X, n et la représentation ----- sont tels que définis précédemment suivi éventuellement de l'action d'un composé de formule R'ₐ-J dans laquelle R'ₐ peut prendre toutes les valeurs de R' et J représente un groupe partant comme un atome d'halogène ou un groupement tosyle, pour obtenir le composé de formule (I/c), cas particulier des composés de formule (I) : 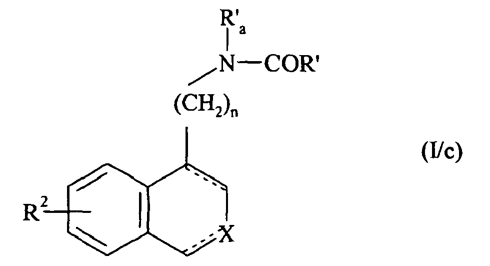 dans laquelle R², R', Rₐ', X, n et la représentation ----- sont tels que définis précédemment,
- soit soumis à l'action d'un composé de formule (IX) :

   O = C = N-R' (IX)

   dans laquelle R' est tel que défini précédemment, suivi éventuellement de l'action d'un composé de formule R'ₐ-J tel que défini précédemment, pour conduire au composé de formule (I/d), cas particuliers des composés de formule (I) : 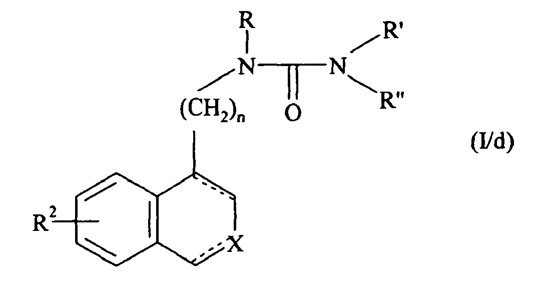
dans laquelle R², R, R', n, X et la représentation ----- sont tels que définis précédemment et R" a la même définition que dans la formule (I), les composés de formule (I/a) à (I/d) pouvant être soumis à un agent de thionation comme le réactif de Lawesson par exemple pour conduire au composé de formule (I/e), cas particulier des composés de formule (I) : 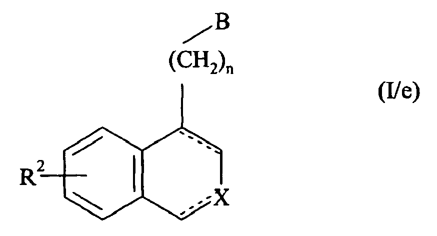 dans laquelle R², n et la représentation ----- sont tels que définis précédemment et B représente un groupement C(S)NRR', N(R)C(S)R' ou N(R)C(S)NR'R" dans lesquels R, R' et R" sont tels que définis précédemment,
les composés (Va) à (I/e) formant l'ensemble des composés de formule (I) et pouvant être purifiés selon une technique classique de séparation, que l'on transforme, si on le souhaite, en leurs sels d'addition à un acide ou à une base pharmaceutiquement acceptable, et dont on sépare éventuellement les isomères selon une technique classique de séparation.

Les composés (II) de départ sont soit commerciaux, soit aisément accessibles à l'homme du métier par des réactions chimiques classiques ou décrites dans la littérature.

Les composé de l'invention et les compositions pharmaceutiques les contenant s'avèrent être utiles pour le traitement des troubles du système mélatoninergique.

L'étude pharmacologique des dérivés de l'invention a en effet montré qu'ils étaient atoxiques, doués d'une haute affinité pour les récepteurs de la mélatonine et possédaient d'importantes activités sur le système nerveux central ainsi que sur la microcirculation qui permettent d'établir que les produits de l'invention sont utiles dans le traitement du stress, des troubles du sommeil, de l'anxiété, de la dépression majeure, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention peuvent être utilisés dans les dysfonctionnements sexuels, qu'ils possèdent des propriétés d'inhibiteurs de l'ovulation, d'immmunomodulateurs et qu'ils sont susceptibles d'être utilisés dans le traitement des cancers.

Les composés seront utilisés de préférence dans les traitements de la dépression majeure, des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières, et des troubles du sommeil.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Les exemples suivants illustrent l'invention et ne la limitent en aucune façon. Les préparations suivantes conduisent à des composés de l'invention ou à des intermédiaires de synthèse utiles dans la préparation des composés de l'invention.

### Préparation 1 : 4-(6-Méthoxy-3,4-dihydro-4-isoquinolinyl)butanoate d'éthyle, chlorhydrate

### Stade A : 5-Cyano-5-(3-méthoxyphényl)pentanoate d'éthyle

Deux grammes de (3-méthoxyphényl)acétonitrile et 1,5 ml de 4-bromobutyrate d'éthyle sont dissous dans 50 ml de diméthylformamide à 0°C. Six cents milligrammes d'hydrure de sodium 60% (600 mg ; 15 mmol) sont ajoutés progressivement à la solution. Le milieu réactionnel est placé sous agitation à température ambiante pendant 4 heures, est repris par 100 ml d'eau acide et extrait à l'éther. La phase organique est ensuite séchée sur sulfate de magnésium, filtrée et évaporée sous pression réduite. L'huile obtenue est purifiée sur colonne (éluant : éther/cyclohexane 4/6) pour conduire au produit du titre sous la forme d'une huile jaune.

### Stade B : 6-Amino-5-(3-méthoxyphényl)hexanoate d'éthyle, chlorhydrate

Le composé obtenu au stade A (11,2 g ; 43 mmol), préalablement dissous dans 150 ml d'éthanol, est versé dans un autoclave, puis le nickel de Raney est ajouté (10% en masse). Le milieu est ensuite mis sous pression d'hydrogène (10 bars) et chauffé sous agitation à 50°C pendant 48 heures. Après filtration du nickel de Raney, la phase organique est évaporée sous pression réduite. Le résidu obtenu est repris par de l'éther, HCl_{(g)} est mis à barboter dans la solution, puis celle-ci est laissée sous agitation jusqu'à précipitation. Le précipité obtenu est ensuite essoré, recristallisé dans le toluène et le produit du titre est obtenu sous la forme d'un solide blanc.
Point de fusion : 104-106°C

### Stade C : 6-(Formylamino)-5-(3-méthoxyphényl)hexanoate d'éthyle

L'amine obtenue au stade B sous forme base (6,4 g ; 27 mmol) est dissoute dans 60 ml de formiate d'éthyle. Le milieu réactionnel est placé sous agitation et chauffé à reflux pendant 6 heures, puis évaporé sous pression réduite. Le résidu obtenu est repris par de l'éther. La phase organique est ensuite successivement lavée avec de l'eau acide (HCl 1N), de l'eau et une solution d'hydrogénocarbonate à 10%, puis séchée sur sulfate de magnésium, filtrée et évaporée sous pression réduite pour conduire au produit du titre sous la forme d'une huile jaune.

### Stade D : 4-(6-Méthoxy-3,4-dihydro-4-isoquinolinyl)butanoate d'éthyle, chlorhydrate

Le formaldéhyde obtenu au stade C (6,8 g ; 23 mmol) est dissous dans 100 ml d'acétonitrile, puis le milieu réactionnel est chauffé à environ 60°C. L'oxychlorure de phosphore (7 ml) est ajouté à la solution, qui est placée sous agitation et chauffée à reflux pendant 6 heures, puis évaporée sous pression réduite. Le résidu obtenu est repris par deux fois par de l'éthanol et évaporé sous pression réduite, puis repris par de l'eau. La phase aqueuse est lavée au dichlorométhane, puis alcalinisée avec une solution saturée d'hydrogénocarbonate de sodium et extraite au dichlorométhane. La phase organique est séchée sur sulfate de magnésium, filtrée et évaporée sous pression réduite. L'huile obtenue est reprise par de l'éther saturée en HCl_{(g)} puis évaporée sous pression réduite. Le résidu est repris dans du toluène à chaud et laissé sous agitation jusqu'à précipitation. Le précipité obtenu est ensuite essoré et le produit du titre est obtenu sous la forme d'un solide blanc.
Point de fusion : 97-99°C

### Préparation 2 : 4-(2-Aminoéthyl)-6-méthoxy-3,4-dihydro-2(1H)-isoquinoline carboxylate de tert-butyle

### Stade A : Cyano(3-méthoxyphényl)acétate de méthyle

Vingt-cinq grammes de (3-méthoxyphényl)acétonitrile sont dissous dans 200 ml de THF anhydre dans un Erlermeyer à col rodé. L'hydrure de sodium à 60% (8,88 g ; 0,37 mol) est ajouté à la solution et le milieu réactionnel est chauffé à reflux sous agitation pendant 30 minutes. Le carbonate de diméthyle (58 ml ; 0,6814 mol) est alors ajouté goutte à goutte sur une demi-heure puis le milieu réactionnel est chauffé à reflux sous agitation pendant 2 heures. Le milieu réactionnel est versé dans de l'eau froide et légèrement acide. La phase aqueuse est extraite à l'éther, puis la phase éthérée est lavée à l'eau avant d'être évaporée. Une solution de carbonate de potassium (47,15 g ; 0,34 mol ) est ajoutée à l'huile obtenue précédemment. Après agitation, le milieu est lavé à l'éther. La phase éthérée obtenue est relavée avec une solution de carbonate potassium (12,02 g ; 0,08 mol). Les deux phases aqueuses sont rassemblées, acidifiées aussitôt et extraites à l'éther. La phase organique ainsi obtenue est lavée avec une solution d'hydrogénocarbonate de sodium à 10%, séchée sur sulfate de magnésium et évaporée sous pression réduite pour conduire au produit du titre sous la forme d'une huile jaune orangée.

### Stade B : 3-Amino-2-(3-méthoxyphényl)propanoate de méthyle, chlorhydrate

Le composé obtenu au stade A (34,32 g ; 0,1672 mol) est dissous dans 150 ml de méthanol. La solution est versée dans un autoclave puis 50 ml de chloroforme et l'oxyde de platine (10% en masse) sont ajoutés à la solution. L'autoclave est mis sous pression d'hydrogène (60 bars) à température ambiante et laissé sous agitation magnétique pendant 24 heures. Après filtration du catalyseur, la solution est évaporée sous pression réduite. L'huile obtenue est reprise par de l'éther. Le précipité formé est essoré et recristallisé dans l'acétonitrile pour conduire au produit du titre sous la forme d'un solide blanc.
Point de fusion : 170-172°C

### Stade C : 3-(Formylamino)-2-(3-méthoxyphényl)propanoate de méthyle

Le composé obtenu au stade B (20,25 g ; 0,08 mol), sous forme base, est dissous dans 130 ml de formiate d'éthyle (1,81 mol). Le milieu réactionnel est chauffé à reflux pendant 6 heures puis évaporé sous pression réduite. L'huile obtenue est reprise par de l'acétate d'éthyle. La phase organique est lavée à l'eau basique (NaHCO₃), séchée sur sulfate de magnésium, filtrée et évaporée pour conduire au produit du titre sous la forme d'une huile jaune.

### Stade D : 6-Méthoxy-3,4-dihydro-4-isoquinolinecarboxylate de méthyle, chlorhydrate

Le composé obtenu au stade C (8,03 g ; 0,03 mol) est dissous dans 100 ml d'acétonitrile, puis le milieu réactionnel est chauffé à environ 60°C. L'oxychlorure de phosphore (16 ml ; 0,17 mol) est ajouté à la solution, celle-ci est placée sous agitation et chauffée à reflux pendant 6 heures, puis évaporée sous pression réduite. Le résidu obtenu est repris par deux fois par du méthanol et évaporé sous pression réduite puis reprise par un minimum d'acétone. Le précipité formé est ensuite essoré pour conduire au produit du titre sous la forme d'un solide blanc.
Point de fusion : 212-215°C

### Stade E : 6-Méthoxy-1,2,3,4-tétrahydro-4-isoquinolinecarboxylate de méthyle, chlorhydrate

Le composé obtenu au stade D (9,21g), sous forme base, est dissous dans 150 ml de méthanol puis le palladium sur charbon (900 mg) est ajouté à la solution. Le milieu réactionnel est placé sous agitation, à température ambiante et sous hydrogène pendant 4 heures. Après filtration du palladium sur charbon, la phase organique est évaporée sous pression réduite. L'huile obtenue est reprise par de l'éther saturée en acide chlorhydrique. Le précipité formé est essoré et recristallisé dans l'acétonitrile pour conduire au produit du titre sous la forme d'un solide blanc.
Point de fusion : 191-193°C

### Stade F : 6-Méthoxy-3,4-dihydro-2,4(1H)-isoquinolinedicarboxylate de 2-tert-butyle et 4-méthyle

Le composé obtenu au stade E (4,02 g ; 15 mmol) est mis en suspension dans 100 ml de dichlorométhane puis la triéthylamine (6,6 ml) est ajoutée. Après solubilisation complète, le di-*tert*-butyl dicarbonate (4 g ; 18 mmol) est ajouté et le milieu réactionnel est laissé sous agitation à température ambiante pendant 30 minutes. La solution est versée dans 100 ml d'eau et l'excès de triéthylamine est neutralisé avec de l'eau acide (HCl 0,1N). Après séparation, la phase aqueuse est extraite au dichlorométhane et les solutions organiques réunies sont séchées sur sulfate de magnésium, filtrées et évaporées sous pression réduite. Le produit du titre est purifié par chromatographie sur gel de silice.
Huile incolore.

### Stade G : 4-(Hydroxyméthyl)-6-méthoxy-3,4-dihydro-2(1H)-isoquinolinecarboxylate de tert-butyle

L'hydrure mixte de lithium et d'aluminium (5,62 g ; 148 mmol) est mis en suspension dans 50 ml de tétrahydrofurane anhydre. Une solution du composé obtenu au stade F (11,9 g ; 37 mmol) préalablement dissoute dans 50 ml de tétrahydrofurane anhydre, est ensuite ajoutée goutte à goutte. Le milieu réactionnel est alors laissé sous agitation à température ambiante pendant 2 heures. Un minimum de soude (NaOH 2N) est ajouté au milieu réactionnel jusqu'à ce qu'il n'y ai plus de dégagement gazeux afin de former les précipités d'hydroxyde de lithium et d'alumine. Ceux-ci sont ensuite filtrés et lavés avec du tétrahydrofurane. La phase organique est évaporée sous pression réduite. Le composé du titre est purifié par chromatographie sur gel de silice.
Huile jaune claire.

### Stade H : 6-Méthoxy-4-{[(méthylsulfonyl)oxy]méthyl}-3,4-dihydro-2(1H)-isoquinoline carboxylate de tert-butyle

Le composé obtenu au stade G (10,5 g; 36 mmol) est solubilisé dans 150 ml de dichlorométhane puis la triéthylamine (8,5 ml) est ajoutée. La solution est refroidie à 0°C et le chlorure de méthane sulfonyle (4,8 ml ; 62 mmol) est ajouté goutte à goutte. Le milieu réactionnel est laissé sous agitation à température ambiante pendant 2 heures, puis versé dans 150 ml d'eau. La solution est extraite au dichlorométhane, séchée sur sulfate de magnésium, filtrée et évaporée sous pression réduite. Le composé du titre est purifié par chromatographie sur gel de silice.
Huile jaune.

### Stade 1 : 4-(Cyanométhyl)-6-méthoxy-3,4-dihydro-2(1H)-isoquinolinecarboxylate de tert-butyle

Le cyanure de potassium (5,52 g ; 85 mmol) est mis en suspension dans 50 ml de DMSO et la solution est chauffée à 80°C. Le composé obtenu au stade H (6,3 g ; 17 mmol), préalablement dissous dans 50 ml de DMSO, est ajouté progressivement à la solution précédente, puis le milieu réactionnel est chauffé encore pendant 30 minutes à 80°C. La solution est versée dans 150 ml d'eau, extraite par trois fois au dichlorométhane. La phase organique est ensuite séchée sur sulfate de magnésium, filtrée et évaporée. L'huile rouge foncée obtenue est purifiée par chromatographie sur gel de silice (éluant : cyclohexane avec ajout progressif d'acétate d'éthyle jusqu'aux proportions 8/2) et le solide obtenu est recristallisé dans le cyclohexane pour conduire au produit du titre sous la forme d'un solide blanc.
Point de fusion : 75-77°C

### Stade J : 4-(2-Aminoéthyl)-6-méthoxy-3,4-dihydro-2(1H)-isoquinolinecarboxylate de tert-butyle

Le composé obtenu au stade I (6,3 g ; 21 mmol) est dissous dans 150 ml de méthanol saturé avec du NH_{3(g)}. La solution est versée dans un autoclave et le nickel de Raney (600mg) est ajouté. Le milieu réactionnel est alors placé sous agitation à 60°C et sous une pression d'hydrogène de 50 bars pendant 6 heures. Après filtration du catalyseur, la solution est évaporée sous pression réduite et le composé du titre est obtenu sous la forme d'une huile incolore.

### Préparation 3 : (6-Méthoxy-4-isoquinolinyl)acétonitrile, chlorhydrate

### Stade A : 6-Méthoxy-4-isoquinolinecarboxylate de méthyle, chlorhydrate

Le composé obtenu au stade D de la Préparation 2 (1,56 g ; 0,006 mol), sous forme base, est dissous dans 10 ml de décahydronaphtalène puis le palladium activé sur charbon est ajouté (10% en masse). Le milieu réactionnel est chauffé à 130 °C sous agitation pendant 24 heures. Le catalyseur est filtré à chaud et lavé à l'acétate d'éthyle. Après évaporation sous pression réduite, l'huile obtenue est reprise par une solution éthérée saturée en HCl_{(g)}. Le précipité formé est essoré et recristallisé dans l'acétonitrile pour conduire au produit du titre sous la forme d'un solide blanc.
Point de fusion : 178-180°C

### Stade B : (6-Méthoxy-4-isoquinolinyl)méthanol, chlorhydrate

Le composé obtenu au stade A (0,395 g ; 0,0015 mol), sous forme base, est dissous dans 200 ml d'éther. L'hydrure mixte de lithium et d'aluminium (0,14 g ; 0,004 mol) est alors ajouté progressivement en refroidissant le ballon dans de la glace. Le milieu réactionnel est laissé sous agitation à température ambiante une semaine. Un minimum de soude 30% (quelques gouttes) est ajouté au milieu réactionnel afin de former les précipités d'hydroxyde de lithium et d'alumine. Ceux-ci sont ensuite filtrés et lavés à l'acétate d'éthyle. La phase organique est séchée sur sulfate de magnésium, filtrée et évaporée sous pression réduite. L'huile obtenue est reprise par de l'éther saturé en HCl_{(g)}. Le précipité formé est essoré et recristallisé dans l'acétonitrile pour conduire au produit du titre sous la forme d'un solide blanc.
Point de fusion : 250-252°C

### Stade C : 4-(Chlorométhyl)-6-méthoxyisoquinoline

Le chlorhydrate du composé obtenu au stade B (1,09 g ; 0,005 mol) est mis en suspension dans 50 ml de chloroforme. Le chlorure de thionyle (2,80 ml ; 0,04 mol) est ajouté puis le milieu réactionnel est chauffé à reflux sous agitation pendant 24 heures. Après évaporation sous pression réduite, le résidu obtenu est repris par de l'éther éthylique. Le précipité formé est essoré et recristallisé dans l'acétonitrile pour conduire au composé du titre sous la forme d'un solide blanc.
Point de fusion: 256-257°C

### Stade D : (6-Méthoxy-4-isoquinolinyl)acétonitrile, chlorhydrate

Le chlorhydrate du composé obtenu au stade C (0,60 g ; 0,0024 mol) est mis en solution dans 10 ml d'une solution aqueuse saturée en carbonate de potassium et 40 ml de dichlorométhane. Le bromure de tétrabutylammonium (2 g ; 0,006 mol) et le cyanure de potassium (0,80 g ; 0,012 mol) sont alors ajoutés à la solution précédente. Le milieu réactionnel est laissé sous agitation à température ambiante pendant 24 heures. La solution est extraite au dichlorométhane, la phase organique est lavée à l'eau, séchée sur sulfate de magnésium, filtrée et évaporée. L'huile obtenue est reprise par de l'acétone et de l'éther saturée en HCl_{(g)}. Le précipité formé est essoré et recristallisé dans le toluène/cyclohexane 5/5 pour conduire au composé du titre sous la forme d'un solide jaune.
Point de fusion : 114-115°C

### Préparation 4 : 4-(Aminométhyl)-6-méthoxy-3,4-dihydro-2(1H)-isoquinoline carboxylate de tert-butyle

### Stade A : 6-Méthoxy-4-{[(méthylsulfonyl)oxy]méthyl}-3,4-dihydro-2(1H)-isoquinoline carboxylate de tert-butyle

On procède comme dans les stades A à H de la Préparation 2.

### Stade B : 4-(Azidométhyl)-6-méthoxy-3,4-dihydro-2(1H)-isoquinoline carboxylate de tert-butyle

L'azoture de sodium (1,12 g) est mis en suspension dans 40 ml de DMF et la solution est chauffée à 80°C. Le composé obtenu au stade A (1,6 g), préalablement dissous dans 10 ml de DMF, est ajouté progressivement à la solution précédente, puis le milieu réactionnel est chauffé encore pendant 2 à 3 heures à 80°C.

La solution est versée dans 150 ml d'eau, extraite par trois fois à l'acétate d'éthyle. La phase organique est ensuite séchée sur sulfate de magnésium, filtrée et évaporée. L'huile rouge foncée obtenue est purifiée par chromatographie sur gel de silice (éluant : cyclohexane avec ajout progressif d'acétate d'éthyle jusqu'aux proportions 5/5) pour conduire au produit du titre sous la forme d'une huile incolore.

### Stade C : 4-(Aminométhyl)-6-méthoxy-3,4-dihydro-2(1H)-isoquinoline carboxylate de tert-butyle

Le composé obtenu au stade B (1,14 g) est dissout dans 100 ml de méthanol puis le palladium sur charbon (120 mg) est ajouté à la solution. Le milieu réactionnel est placé sous agitation, à température ambiante et sous hydrogène pendant 3 heures.
Après filtration du palladium sur charbon, la phase organique est évaporée sous pression réduite. Le résidu obtenu est purifié par chromatographie sur gel de silice (éluant : cyclohexane avec ajout progressif d'acétate d'éthyle jusqu'aux proportions 5/5) pour conduire au produit du titre sous la forme d'une huile incolore.

### Exemple 1 : 4-(6-méthoxy-3,4-dihydro-4-isoquinolinyl)-N-méthylbutanamide

Le composé obtenu dans la Préparation 1 (41 mmol) sous forme base est mis en solution dans 10 ml d'éthanol. 60 ml de méthylamine aqueuse sont ajoutés et le milieu réactionnel est placé sous agitation à température ambiante pendant 12 heures. Après évaporation sous pression réduite, le filtrat est repris par 50 ml d'eau et extrait au dichlorométhane. La phase organique est lavée à l'eau, séchée sur sulfate de magnésium, filtrée et évaporée sous pression réduite. Le produit du titre est obtenu après chromatographie de l'huile obtenue sur gel de silice.
Huile jaune.

### Exemple 2 : 4-(6-Méthoxy-1,2,3,4-tétrahydro-4-isoquinolinyl)-N-méthylbutanamide

Le composé obtenu dans l'Exemple 1 (54 mmol), sous forme base, est dissoute dans 50 ml de méthanol puis le palladium sur charbon (150 mg) est ajouté à la solution. Le milieu réactionnel est placé sous agitation, à température ambiante et sous hydrogène pendant 4 heures. Après filtration du palladium sur charbon, la phase organique est évaporée sous pression réduite. L'huile obtenue est purifiée par chromatographie sur gel de silice pour conduire au produit du titre sous la forme d'une huile jaune.

### Exemple 3 : 4-(6-méthoxy-4-isoquinolinyl)-N-méthylbutanamide

### Stade A : 4-(6-Méthoxy-4-isoquinolinyl)butanoate d'éthyle, chlorhydrate

Le composé obtenu dans la Préparation 1 (1,6 g ; 5 mmol) est dissoute à chaud dans 20 ml de toluène contenant de la triéthylamine (0,9 ml) et de l'éthanol absolu (2 ml). Le palladium sur charbon (300 mg) est ajouté au milieu réactionnel, puis celui-ci est placé sous agitation et chauffé à reflux pendant 24 heures. Après filtration du palladium sur charbon, la phase organique est évaporée sous pression réduite. Le résidu obtenu est purifié par chromatographie sur gel de silice (éluant : dichlorométhane avec ajout progressif de méthanol). L'huile obtenue est mise sous forme de chlorhydrate puis le précipité formé est recristallisé dans l'acétonitrile.
Point de fusion : 190-192°C

### Stade B : 4-(6-méthoxy-4-isoquinolinyl)-N-méthylbutanamide

Le composé obtenu dans le stade A (2,2 g ; 7 mmol) est mis en solution dans 30 ml de méthylamine (40% dans l'eau) et le mélange est chauffé à reflux sous agitation pendant 3 heures. Le milieu réactionnel est repris par 50 ml d'eau et extrait à l'éther. La phase organique est lavée à l'eau, séchée sur sulfate de magnésium, filtrée et évaporée sous pression réduite. L'huile obtenue est purifiée par chromatographie sur gel de silice (éluant : dichlorométhane avec ajout progressif de méthanol). L'huile obtenue est évaporée sous pression réduite et le précipité ainsi formé est recristallisé dans le toluène pour conduire au produit du titre sous la forme d'un solide blanc.
Point de fusion : 117-119°C

### Microanalyse élémentaire :

| | | | |
|---|---|---|---|
| | C% | H% | N% |
| *Calculé* | 69,74 | 7,02 | 10,84 |
| *Trouvé* | 69,64 | 7,22 | 10,83 |

### Exemple 4 : N-[2-(6-méthoxy-1,2,3,4-tétrahydro-4-isoquinolinyl)éthyl]acétamide, chlorhydrate

Le composé obtenu au stade I de la Préparation 2 (6 g ; 20 mmol) est solubilisé dans 100 ml d'anhydride acétique puis la solution est versée dans un autoclave. Le nickel de Raney (600 mg) est alors ajouté à la solution et le milieu réactionnel est placé sous agitation à 60°C et sous pression d'hydrogène de 50 bars pendant 6 heures. Après filtration du catalyseur, la solution est évaporée sous pression réduite. Le résidu orange obtenu est repris par une solution de soude à 10% et laissé sous agitation pendant 15 minutes, puis extrait à l'acétate d'éthyle (3 fois). La phase organique est lavée une fois à l'eau, séchée sur sulfate de magnésium, filtrée et évaporée sous pression réduite. L'huile obtenue est reprise par 50 ml de méthanol. De l'acide chlorhydrique gazeux est mis à barboter dans la solution puis celle-ci est laissée sous agitation avec une garde à CaCl₂ pendant 24 heures. La solution est évaporée sous pression réduite et le précipité ainsi obtenu est recristallisé dans l'acétonitrile pour conduire au produit du titre sous la forme d'un solide blanc.
Point de fusion : 182-184°C

### Exemple 5 : N-[2-(6-méthoxy-1,2,3,4-tétrahydro-4-isoquinolinyl)éthyl]propanamide, chlorhydrate

Le composé obtenu dans la Préparation 2 et 2 équivalents de carbonate de potassium sont dissous dans un mélange acétate d'éthyle/eau 3/2. Le chlorure de propanoyle (2 équivalents) est alors additionné goutte à goutte à la solution. Le milieu réactionnel est laissé sous agitation à température ambiante pendant une heure. Après séparation des phases, la phase organique est lavée à l'eau, séchée sur sulfate de magnésium, filtrée et évaporée sous pression réduite. L'huile obtenue est purifiée par chromatographie sur gel de silice (éluant acétate d'éthyle/cyclohexane 5/5). L'huile obtenue est reprise par du méthanol. De l'acide chlorhydrique gaz est mis à barboter dans la solution puis celle-ci est laissée sous agitation avec une garde à CaCl₂ pendant 24 heures. La solution est évaporée sous pression réduite et le composé du titre est obtenu sous la forme d'un solide blanc.
Point de fusion: 161-163°C

### Exemple 6 : N-[2-(6-méthoxy-1,2,3,4-tétrahydro-4-isoquinolinyl)éthyl]butanamide, chlorhydrate

Le composé du titre est obtenu par le même procédé que dans l'Exemple 5 en remplaçant le chlorure de propanoyle par le chlorure de butanoyle.
Solide blanc très hygroscopique.

### Exemple 7 : N-[2-(6-méthoxy-1 ,2,3,4-tétrahydro-4-isoquinolinyl)éthyl]cyclopropane carboxamide, chlorhydrate

Le composé du titre est obtenu par le même procédé que dans l'Exemple 5 en remplaçant le chlorure de propanoyle par le chlorure de l'acide cyclopropyle carboxylique.
Solide blanc.
Point de fusion : 215-217°C

### Exemple 8 : N-[2-(6-méthoxy-1,2,3,4-tétrahydro-4-isoquinolinyl)éthyl]cyclobutane carboxamide, chlorhydrate

Le composé du titre est obtenu par le même procédé que dans l'Exemple 5 en remplaçant le chlorure de propanoyle par le chlorure de l'acide cyclobutyle carboxylique.
Solide blanc.
Point de fusion : 130-132°C

### Exemple 9 : N-[2-(6-méthoxy-4-isoquinolinyl)éthyl]acétamide, chlorhydrate

Le composé obtenu dans la Préparation 3 (0,30 g ; 0,0015 mol), sous forme base, préalablement dissous dans 10 ml d'anhydride acétique, est versé dans un autoclave, puis le nickel de Raney est ajouté (10% en masse). Le milieu est ensuite mis sous pression d'hydrogène (60 bars) et chauffé sous agitation à 60°C pendant 6 heures. Après filtration du nickel de Raney, la phase organique est reprise par de la soude à 10% à température ambiante et laisser sous agitation magnétique pendant 15 minutes. La solution est extraite à l'acétate d'éthyle, la phase organique est lavée à la saumure, séchée sur sulfate de magnésium, filtrée et évaporée sous pression réduite. L'huile obtenue est purifiée sur colonne (éluant dichlorométhane avec ajoutant progressif de méthanol jusqu'aux proportions 9/1). L'huile purifiée est reprise par de l'éther saturé en HCl_{(g)}. Le précipité formé est essoré et recristallisé dans l'éthanol pour conduire au produit du titre sous la forme d'un solide blanc.
Point de fusion : 212-214°C

### Exemple 10 : N-[2-(6-méthoxy-4-isoquinolinyl)éthyl]propanamide, chlorhydrate

Le composé de l'Exemple 5 est solubilisé dans un minimum de méthanol à chaud puis dilué dans du toluène. 1,5 équivalents de triéthylamine sont alors ajoutés à la solution puis le milieu réactionnel est chauffé à reflux en présence de palladium sur charbon (10% en masse) pendant 3 heures. Après filtration du catalyseur, la solution est évaporée sous pression réduite. L'huile obtenue est purifiée par chromatographie sur gel de silice (éluant dichlorméthane/méthanol 9/1). L'huile purifiée est reprise par de l'éther saturé en HCl_{(g)} et laissé sous agitation jusqu'à obtention d'un précipité. Le précipité ainsi formé est essoré et placé sous vide dans un dessicateur. Le produit du titre est obtenu sous la forme d'un solide blanc.
Point de fusion : 233-235°C

### Exemple 11 : N-[2-(6-méthoxy-4-isoquinolinyl)éthyl]butanamide, chlorhydrate

Le composé du titre est obtenu selon le même procédé que dans l'Exemple 10 à partir du composé obtenu dans l'Exemple 6.
Point de fusion : 195-197°C

### Exemple 12 : N-[2-(6-méthoxy-4-isoquinolinyl)éthyl]cyclopropanecarboxamide chlorhydrate

Le composé du titre est obtenu selon le même procédé que dans l'Exemple 10 à partir du composé obtenu dans l'Exemple 7.
Point de fusion : 226-228°C

### Exemple 13 : N-[2-(6-méthoxy-2-phényl-1,2,3,4-tétrahydro-4-isoquinolinyl)éthyl] acétamide, chlorhydrate

Le composé obtenu dans l'Exemple 4 (460 mg ; 1,8 mmol) sous forme base est mis en suspension dans 30 ml de dichlorométhane. Le triphénylbismuth (900 mg ; 2 mmol) et l'acétate de cuivre Cu(OAc)₂ (190 mg ; 0,95 mmol) sont alors ajoutés. Le milieu réactionnel est placé sous argon et laissé sous agitation magnétique à température ambiante pendant 18 heures. Le milieu est filtré, repris par de l'eau et extrait par l'acétate d'éthyle. La phase organique est séchée sur sulfate de magnésium, filtrée et évaporée sous pression réduite. Le résidu obtenu est purifié par chromatographie sur gel de silice (éluant acétate d'éthyle/cyclohexane 2/8). L'huile purifiée est reprise par de l'éther saturée en HCI(g) et laissée sous agitation jusqu'à obtention d'un précipité, qui est alors essoré et placé sous vide dans un dessicateur pour conduire au produit du titre sous la forme d'un solide blanc.
Point de fusion : 83-85°C

### Exemple 14 : N-[2-(2-benzyl-6-méthoxy-1,2,3,4-tétrahydro-4-isoquinolinyl)éthyl] acétamide

Le composé obtenu dans l'Exemple 4 (740 mg ; 2,5 mmol) et le carbonate de potassium (720 mg ; 5 mmol) sont mis en suspension dans 20 ml de DMF, puis le bromure de benzyle (0,37 ml ; 3 mmol) est ajouté à la solution. Le milieu réactionnel est chauffé à 125°C sous agitation pendant 4 heures. La solution est versée dans 50 ml d'eau, acidifiée par HCI 6N et lavée à l'acétate d'éthyle. La phase aqueuse est ensuite alcalinisée par du carbonate de potassium et extraite par du dichlorométhane. La phase organique est séchée sur sulfate de magnésium, filtrée et évaporée sous pression réduite. Le résidu ainsi obtenu est purifié par chromatographie sur gel de silice (éluant : dichlorométhane avec ajout progressif de méthanol jusqu'aux proportions 9/1). L'huile purifiée précipite. Le précipité ainsi formé est recristallisé dans un mélange toluène/cyclohexane 7/3 pour conduire au produit du titre sous la forme d'un solide blanc.
Point de fusion : 123-125°C

### Exemple 15 : N-{2-[2-(3-formylphényl)-6-méthoxy-1,2,3,4-tétrahydro-4-isoquinolinyl] éthyl}acétamide

L'acétate de cuivre Cu(OAc)₂ (960 mg ; 5 mmol) et la triéthylamine (1,5 ml ; 10,5 mmol) sont mis en suspension dans 60 ml de dichlorométhane. Le composé de l'Exemple 4 (1 g ; 3,5 mmol), l'acide 3-formylphénylboronique (1,05 g ; 7 mmol) et du tamis moléculaire sont alors ajoutés successivement et progressivement à la solution. Le milieu réactionnel est laissé sous agitation à température ambiante pendant 2 heures. Le milieu est filtré, lavé à l'eau, séché sur sulfate de magnésium, filtré et évaporé sous pression réduite. Le résidu obtenu est purifiée par chromatographie sur gel de silice (éluant : dichlorométhane avec ajout progressif de méthanol jusqu'aux proportions 9/1) pour conduire au produit du titre sous la forme d'une huile jaune.

### Exemple 16 : N-[2-(6-méthoxy-2-méthyl-1,2,3,4-tétrahydro-4-isoquinolinyl)éthyl] acétamide, chlorhydrate

L'acide formique (0,55 ml) et le formaldéhyde à 37 % (0,6 ml) sont ajoutés à 0°C au composé obtenu dans l'Exemple 4 (1,8 g ; 7,2 mmol) sous forme base. Le milieu réactionnel est chauffé à 80°C sous agitation pendant 24 heures. Après avoir refroidi le milieu réactionnel à 0°C, 10 ml d'HCl 6N sont alors ajoutés. Le milieu est ensuite lavé par de l'éther, alcalinisé par NaOH 2N et extrait à l'éther. La phase organique est séchée sur sulfate de magnésium, filtrée et évaporée sous pression réduite. L'huile jaune ainsi obtenue est purifiée par chromatographie sur gel de silice (éluant : dichlorométhane avec ajout progressif de méthanol jusqu'aux proportions 9/1). L'huile purifiée est reprise par de l'éther saturée en HCl_{(g)} et laissée sous agitation jusqu'à obtention d'un précipité qui est alors essoré et placé sous vide dans un dessicateur pour conduire au produit du titre sous la forme d'un solide blanc très hygroscopique.
Point de fusion : 59-61°C

### Exemple 17 : N-{2-[2-Cyclopropylméthyl)-6-méthoxy-1,2,3,4-tétrahydro-4-isoquinolinyl]éthyl}acétamide, chlorhydrate

Le composé obtenu dans l'Exemple 4 (1,03 g) et le carbonate de potassium (1,25 g) sont mis en suspension dans 50 ml d'acétone. La solution est laissée sous agitation à température ambiante pendant 10 minutes, puis le bromure de méthylcyclopropane (0,36 ml) est ajouté à la solution. Le milieu réactionnel est laissé sous agitation à température ambiante pendant 12 heures.
Le carbonate de potassium est filtré et la solution récupérée évaporée. Le résidu est repris sur l'eau et la phase aqueuse est extraite par de l'éther. La phase organique est séchée sur sulfate de magnésium, filtrée et évaporée sous pression réduite. Le résidu ainsi obtenu est purifié par chromatographie sur gel de silice (éluant : dichlorométhane avec ajout progressif de méthanol jusqu'aux proportions 9/1).
L'huile purifiée est reprise par de l'éther saturé en HCI(g) et laissée sous agitation jusqu'à obtention d'un précipité qui est alors essoré et placé sous vide dans un dessicateur pour conduire au produit du titre sous la forme d'un solide blanc hygroscopique.
Point de fusion : < 50°C

### Exemple 18 : N-[(6-Méthoxy-1,2,3,4-tétrahydro-4-isoquinolinyl)méthyl]acétamide, chlorhydrate

Le composé obtenu dans la Préparation 4 (0,77 g) et le carbonate de potassium (1,8 g) sont dissous dans 50 ml d'un mélange dichlorométhane/eau 1/1. Le chlorure d'acétyle (0,47 ml) est alors additionné goutte à goutte à la solution. Le milieu réactionnel est laissé sous agitation à température ambiante pendant 2 heures.
Après séparation des phases, la phase organique est lavée à l'eau, séchée sur sulfate de magnésium, filtrée et évaporée sous pression réduite.
L'huile obtenue est reprise par du méthanol. De l'acide chlorhydrique gaz est mis à barboter dans la solution puis celle-ci est laissée sous agitation avec une garde à CaCl₂ pendant 24 heures, le temps de la déprotection. La solution est évaporée sous pression réduite et le précipité obtenu est recristallisé dans l'acétronile pour conduire au produit du titre sous la forme d'un solide blanc.
Point de fusion : 246-248°C

### ETUDE PHARMACOLOGIQUE

### EXEMPLE A: Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Test de la nage forcée

Les composés de l'invention sont testés dans un modèle comportemental, le test de la nage forcée.
L'appareil est constitué d'un cylindre en plexiglas rempli d'eau. Les animaux sont testés individuellement pendant une session de 6 minutes. Au début de chaque test, l'animal est placé au centre du cylindre. Le temps d'immobilisation est enregistré. Chaque animal est jugé immobile quand il cesse de se débattre, et reste à la surface de l'eau, immobile, ne faisant seulement que les mouvements lui permettant de maintenir la tête hors de l'eau.
Après administration 40 minutes avant le début du test, les composés de l'invention diminuent de façon significative la durée d'immobilisation ce qui montre l'activité antidépressive des dérivés de l'invention. En particulier, le composé de l'Exemple 9 administré à 2,5 mg/kg per os fait passer la durée d'immobilisation de 102 secondes (contrôle) à 57 secondes. Le composé de l'Exemple 3 administré à 25 mg/kg per os fait passer la durée d'immobilisation de 129 secondes (contrôle) à 60 secondes.

### EXEMPLE C : Etude de liaison aux récepteurs MT₁ et MT₂ de la mélatonine

Les expériences de liaison aux récepteurs MT₁ ou MT₂ sont réalisées en utilisant la 2-[¹²⁵I]-iodomélatonine comme radioligand de référence. La radioactivité retenue est déterminée à l'aide d'un compteur à scintillation liquide.

Des expériences de liaison compétitive sont ensuite réalisées en triple, avec les différents composés à tester. Une gamme de concentrations différentes est testée pour chaque composé. Les résultats permettent de déterminer les affinités de liaison des composés testés (Kᵢ).

Ainsi, les valeurs des Kᵢ trouvés pour les composés de l'invention attestent d'une liaison pour l'un ou l'autre des sous-types réceptoriels MT₁ ou MT₂, ces valeurs étant ≤ 10 µM. En particulier le composé de l'Exemple 9 a un Kᵢ (MT₁) de 9,12.10⁻⁹M et un Kᵢ (MT₂) de 2,16.10⁻⁹M ; le composé de l'Exemple 3 a un Kᵢ (MT₁) de 3,8.10⁻⁹M et un Kᵢ (MT₂) de 2,6.10⁻⁹M ; le composé de l'Exemple 10 a un Kᵢ (MT₁) de 4,26.10⁻⁹M et un Kᵢ (MT₂) de 1,14.10⁻⁹M.

### EXEMPLE D : Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### Protocole expérimental

Des rats mâles âgés de un mois sont soumis dès leur arrivée au laboratoire à un cycle lumineux de 12h de lumière par 24h (LD 12 : 12).
Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats

Il apparaît clairement que les composés de l'invention permettent d'agir de façon puissante sur le rythme circadien *via* le système mélatoninergique.

### EXEMPLE E : Test des cages claires/obscures

Les composés de l'invention sont testés dans un modèle comportemental, le test des cages claires/obscures, qui permet de révéler l'activité anxiolytique des molécules.

L'appareil est composé de deux boîtes en polyvinyle couvertes de Plexiglas. L'une de ces boîtes est obscure. Une lampe est placée au-dessus de l'autre boîte donnant une intensité lumineuse au centre de celle-ci d'approximativement 4000 lux. Un tunnel opaque en plastique sépare la boîte claire de la boîte sombre. Les animaux sont testés individuellement pendant une session de 5 min. Le plancher de chaque boîte est nettoyé entre chaque session. Au début de chaque test, la souris est placée dans le tunnel, face à la boîte sombre. Le temps passé par la souris dans la boîte éclairée et le nombre de transitions à travers le tunnel sont enregistrés après la première entrée dans la boîte sombre.

Après administration des composés 30 min avant le début du test, les composés de l'invention augmentent de façon significative le temps passé dans la cage éclairée ainsi que le nombre des transitions, ce qui montre l'activité anxiolytique des dérivés de l'invention.

### EXEMPLE F : Activité des composés de l'invention sur l'artère caudale de rat

Les composés de l'invention ont été testés *in vitro* sur l'artère caudale de rat. Les récepteurs mélatoninergiques sont présents sur ces vaisseaux ce qui en fait un modèle pharmacologique relevant pour étudier l'activité de ligands mélatoninergiques. La stimulation des récepteurs peut induire soit une vasoconstriction soit une dilatation en fonction du segment artériel étudié.

### Protocole

Des rats âgés de 1 mois sont habitués pendant 2 à 3 semaines à un cycle lumière/obscurité 12h/12h.
Après sacrifice, l'artère caudale est isolée et maintenue dans un milieu richement oxygéné. Les artères sont ensuite canulées aux deux extrémités, suspendues verticalement dans une chambre d'organe dans un milieu approprié et perfusées via leur extrémité proximale. Les changements de pression dans le débit de la perfusion permettent d'évaluer l'effet vasoconstricteur ou vasodilatateur des composés.
L'activité des composés est évaluée sur des segments pré-contractés par la phényléphrine (1 µM). Une courbe concentration-réponse est déterminée de façon non-cumulative par addition d'une concentration du composé étudié sur le segment pré-contracté. Lorsque l'effet observé a atteint l'équilibre, le milieu est changé et la préparation laissée 20 minutes avant l'addition d'une même concentration de phényléphrine et d'une nouvelle concentration du composé étudié.

### Résultats

Les composés de l'invention modifient de façon significative le diamètre des artères caudales préconstrictées par la phényléphrine.

### EXEMPLE G : Composition pharmaceutique : Comprimés

| | |
|---|---|
| 1000 comprimés dosés à 5 mg de *N*-[2-(6-méthoxy-4-isoquinolinyl)éthyl]acétamide, chlorhydrate (Exemple 9) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composé de formule (I) : 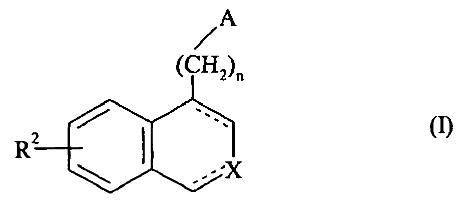 dans laquelle :
◆ n vaut 1, 2 ou 3,
◆ A représente un groupement 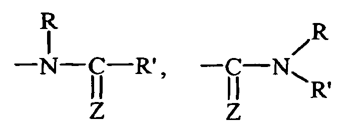 ou un groupement 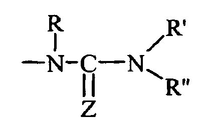 dans lesquels :
• Z représente un atome de soufre ou d'oxygène,
• R et R", identiques ou différents, représentent chacun un atome d'hydrogène ou un groupement alkyle (C₁-C₆) linéaire ou ramifié,
• et R' représente un groupement alkyle (C₁-C₆) linéaire ou ramifié, alkényle (C₂-C₆) linéaire ou ramifié, alkynyle (C₂-C₆) linéaire ou ramifié, cycloalkyle (C₃-C₈), cycloalkyl(C₃-C₈)alkyle(C₁-C₆) dont la partie alkyle est linéaire ou ramifiée, aryle, arylalkyle (C₁-C₆) dont la partie alkyle est linéaire ou ramifiée, hétéroaryle ou hétéroarylalkyle (C₁-C₆) dont la partie alkyle est linéaire ou ramifiée,
◆ X représente un atome d'azote ou un groupement N-R¹ dans lequel R¹ représente un atome d'hydrogène ou un groupement alkyle (C₁-C₆) linéaire ou ramifié, cycloalkyle (C₃-C₈), cycloalkyle (C₃-C₈) alkyle (C₁-C₆) linéaire ou ramifié, aryle, aroyle ou arylalkyle (C₁-C₆) dont la partie alkyle est linéaire ou ramifiée, hétéroaryle, hétéroaroyle ou hétéroarylalkyle (C₁-C₆) dont la partie alkyle est linéaire ou ramifiée,
R² représente un groupement alkoxy (C₁-C₆) linéaire ou ramifié, cycloalkyloxy (C₃-C₈) ou cycloalkyl (C₃-C₈) alkyloxy (C₁-C₆) linéaire ou ramifié,
◆ la représentation ----- signifie que la liaison est simple ou double étant entendu que la valence des atomes est respectée,
étant entendu que :
- par "aryle", on entend un groupement phényle, ou naphtyle, ces groupements étant non substitués ou substitués par un à trois groupements identiques ou différents, choisis parmi alkyle (C₁-C₆) linéaire ou ramifié, alkoxy (C₁-C₆) linéaire ou ramifié, OH, COOH, alkoxycarbonyle dont la partie alkoxy est linéaire ou ramifiée, formyle, nitro, cyano, hydroxyméthyle, amino (éventuellement substitué par un ou deux groupements alkyle (C₁-C₆) linéaire ou ramifié), ou atomes d'halogène,
- par "hétéroaryle", on entend tout groupement mono ou bi-cyclique contenant 5 à 10 chaînons et pouvant contenir 1 à 3 hétéroatomes choisis parmi oxygène, soufre ou azote comme les groupements furane, thiophène, pyrrole, imidazoline, pyridine, quinoleine, isoquinoléine, chromane, indole, benzothiophène ou benzofurane, ces groupements pouvant être partiellement hydrogénés, non substitués ou substitués par un à trois groupements identiques ou différents, choisis parmi alkyle (C₁-C₆) linéaire ou ramifié, alkoxy (C₁-C₆) linéaire ou ramifié, OH, COOH, alkoxycarbonyle dont la partie alkoxy est linéaire ou ramifiée, formyle, nitro, cyano, amino (éventuellement substitué par un ou deux groupements alkyle (C₁-C₆) linéaire ou ramifié), hydroxyméthyle ou atomes d'halogène,
leurs énantiomères et diastéréoisomères, ainsi que leurs sels d'addition à un acide ou à une base pharmaceutiquement acceptable.

2. Composés de formule (I) selon la revendication 1 pour lesquels n vaut 2 et A représente un groupement -NHCOR', leurs énantiomères et diastéréoisomères, ainsi que leurs sels d'addition à un acide ou à une base pharmaceutiquement acceptable.

3. Composés de formule (I) selon la revendication 1 pour lesquels n vaut 3 et A représente un groupement -CONHR', leurs énantiomères et diastéréoisomères, ainsi que leurs sels d'addition à un acide ou à une base pharmaceutiquement acceptable.

4. Composés de formule (I) selon la revendication 1 pour lesquels R² représente un groupement méthoxy, leurs énantiomères et diastéréoisomères, ainsi que leurs sels d'addition à un acide ou à une base pharmaceutiquement acceptable.

5. Composés de formule (I) selon la revendication 1 pour lesquels X représente un atome d'azote, leurs énantiomères et diastéréoisomères, ainsi que leurs sels d'addition à un acide ou à une base pharmaceutiquement acceptable.

6. Composés de formule (I) selon la revendication 1 pour lesquels X représente un groupement NPh ou NBz, leurs énantiomères et diastéréoisomères, ainsi que leurs sels d'addition à un acide ou à une base pharmaceutiquement acceptable.

7. Composé de formule (I) selon la revendication 1 qui est le N-[2-(6-méthoxy-4-isoquinolinyl)éthyl]acétamide ainsi que ses sels d'addition à un acide pharmaceutiquement acceptable.

8. Composé de formule (I) selon la revendication 1 qui est le N-[2-(6-méthoxy-4-isoquinolinyl)éthyl]butanamide ainsi que ses sels d'addition à un acide pharmaceutiquement acceptable.

9. Composé de formule (I) selon la revendication 1 qui est le N-[2-(6-méthoxy-4-isoquinolinyl)éthyl]propanamide ainsi que ses sels d'addition à un acide pharmaceutiquement acceptable.

10. Composé de formule (I) selon la revendication 1 qui est le N-[2-(6-méthoxy-4-isoquinolinyl)éthyl]cyclopropanecarboxamide ainsi que ses sels d'addition à un acide pharmaceutiquement acceptable.

11. Composé de formule (I) selon la revendication 1 qui est le 4-(6-méthoxy-4-isoquinolinyl)-*N*-méthylbutanamide ainsi que ses sels d'addition à un acide pharmaceutiquement acceptable.

12. Composé de formule (I) selon la revendication 1 qui est le N-[2-(6-méthoxy-2-phényl-1,2,3,4-tétrahydro-4-isoquinolinyl)éthyl]acétamide ainsi que ses sels d'addition à un acide pharmaceutiquement acceptable.

13. Composé de formule (I) selon la revendication 1 qui est le N-[2-(2-benzyl-6-méthoxy-1,2,3,4-tétrahydro-4-isoquinolinyl)éthyl]acétamide ainsi que ses sels d'addition à un acide pharmaceutiquement acceptable.

14. Composé de formule (I) selon la revendication 1 qui est le N-{2-[2-(cyclopropylméthyl)-6-méthoxy-1,2,3,4-tétrahydro-4-isoquinolinyl]éthyl}acétamide. ainsi que ses sels d'addition à un acide pharmaceutiquement acceptable.

15. Procédé de préparation de composés de formule (I) selon la revendication 1 **caractérisé en ce que** l'on utilise comme produit de départ le composé de formule (II) : 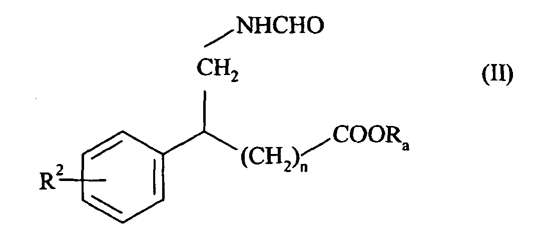 dans laquelle R² et n sont tels que définis dans la formule (I), et Rₐ représente un groupement alkyle (C₁-C₆) linéaire ou ramifié, que l'on soumet à l'action de POCl₃ pour obtenir le composé de formule (III) : 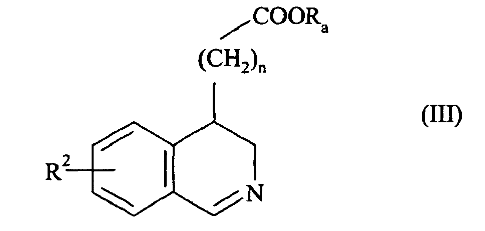 dans laquelle R², n et Rₐ sont définis comme précédemment, que l'on place :
- en présence de palladium sur charbon pour obtenir le composé de formule (IV) : 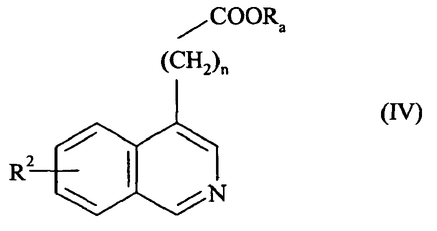
dans laquelle R², n et Rₐ sont définis comme précédemment,
- ou que l'on hydrogène en présence de palladium sur charbon pour conduire au composé et formule (V) : 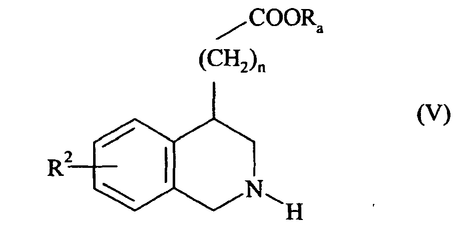
dans laquelle R², n et Rₐ sont définis comme précédemment,
composé de formule (V) sur lequel on condense un composé de formule G-R'¹ dans laquelle G représente un groupe partant et R'¹ peut prendre toutes les valeurs de R¹ à l'exception de l'atome d'hydrogène pour conduire au composé de formule (VI) : 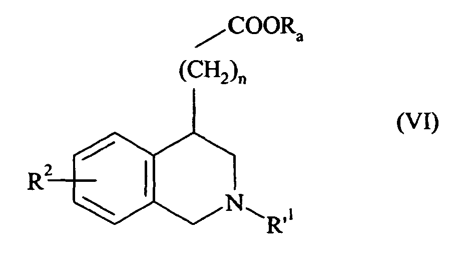 dans lequel R², R'¹, n et Rₐ sont tels que définis précédemment, les composés de formule (III) à (VI) formant le composé de formule (VII) : 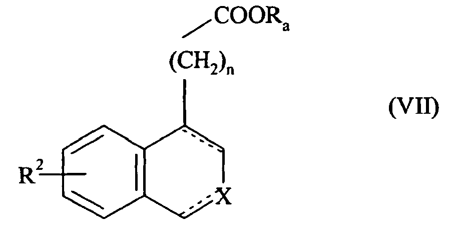 dans laquelle R², n et Rₐ sont tels que définis précédemment et X et la représentation ----- ont la même définition que dans la formule (I),
sur lequel on condense une amine de formule HNRR' dans laquelle R et R' ont la même définition que dans la formule (I) pour obtenir un composé de formule (I/a) cas particulier des composés de formule (I) : 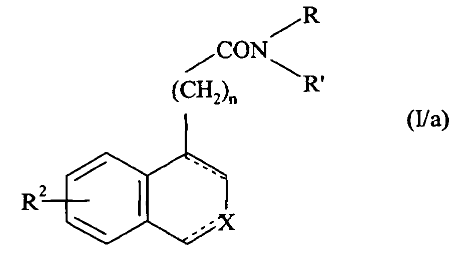 dans laquelle R², R, R', X, n et la représentation ----- sont définis comme précédemment,
ou composé de formule (VII) qui est soumis à une suite réactionnelle classique en chimie organique pour conduire au composé de formule (VIII) : 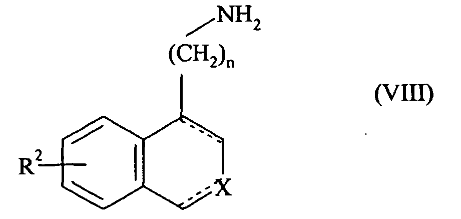 dans lequel R², X, n et la représentation ----- sont tels que définis précédemment, qui est :
- soit mis en réaction avec un chlorure d'acyle ClCOR' ou l'anhydride mixte ou symétrique correspondant pour lesquels R' est tel que défini précédemment pour conduire au composé de formule (I/b), cas particulier du composé de formule (I) : 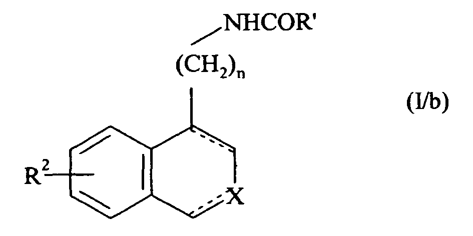
dans laquelle R², R', X, n et la représentation ----- sont tels que définis précédemment suivi éventuellement de l'action d'un composé de formule R'ₐ-J dans laquelle R'ₐ peut prendre toutes les valeurs de R' et J représente un groupe partant comme un atome d'halogène ou un groupement tosyle, pour obtenir le composé de formule (I/c), cas particulier des composés de formule (I) : 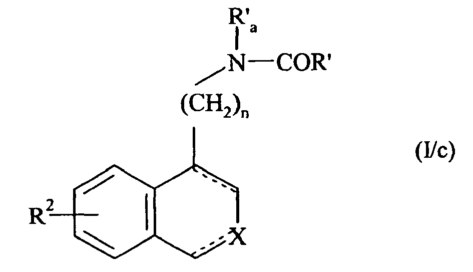 dans laquelle R², R', Rₐ', X, n et la représentation ----- sont tels que définis précédemment,
- soit soumis à l'action d'un composé de formule (IX) :
O = C = N-R' (IX)
dans laquelle R' est tel que défini précédemment, suivi éventuellement de l'action d'un composé de formule R'ₐ-J tel que défini précédemment, pour conduire au composé de formule (I/d), cas particuliers des composés de formule (I) : 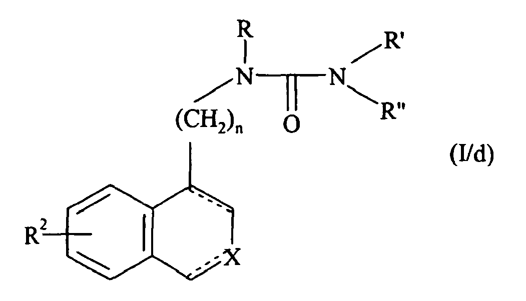 dans laquelle R², R, R', n, X et la représentation ----- sont tels que définis précédemment et R" a la même définition que dans la formule (I), les composés de formule (Va) à (I/d) pouvant être soumis à un agent de thionation comme le réactif de Lawesson par exemple pour conduire au composé de formule (I/e), cas particulier des composés de formule (I) : 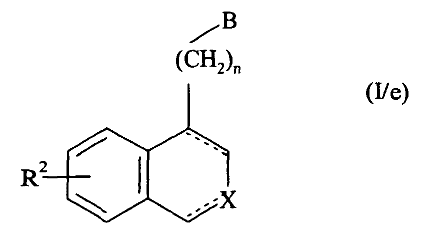 dans laquelle R², n et la représentation ----- sont tels que définis précédemment et B représente un groupement C(S)NRR', N(R)C(S)R' ou N(R)C(S)NR'R" dans lesquels R, R' et R" sont tels que définis précédemment,
les composés (I/a) à (I/e) formant l'ensemble des composés de formule (I) et pouvant être purifiés selon une technique classique de séparation, que l'on transforme, si on le souhaite, en leurs sels d'addition à un acide ou à une base pharmaceutiquement acceptable, et dont on sépare éventuellement les isomères selon une technique classique de séparation.

16. Compositions pharmaceutiques contenant les composés de formule (I) selon l'une quelconque des revendications 1 à 14 ou un de leurs sels d'addition avec un acide ou une base pharmaceutiquement acceptable en combinaison avec un ou plusieurs excipients pharmaceutiquement acceptables.

17. Compositions pharmaceutiques selon la revendication 16 utiles pour la fabrication de médicaments pour traiter les troubles du système mélatoninergique.

18. Compositions pharmaceutiques selon la revendication 16 utiles pour la fabrication de médicaments pour le traitement des troubles du sommeil, du stress, de l'anxiété, des dépressions saisonnières ou de la dépression majeure, des pathologies cardiovasculaires, des pathologies du système digestif, des insomnies et fatigues dues aux décalages horaires, de la schizophrénie, des attaques de paniques, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, des troubles de la circulation cérébrale, ainsi que dans les dysfonctionnements sexuels, en tant qu'inhibiteurs de l'ovulation, d'immunomodulateurs et dans le traitement des cancers.

## Claims

1. Compound of formula (I) : 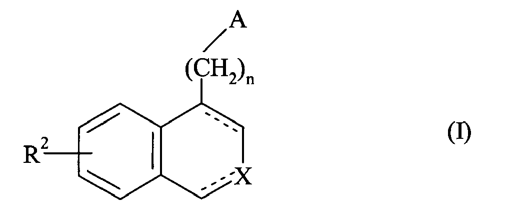 wherein:
◆ n is 1, 2 or 3,
◆ A represents a group 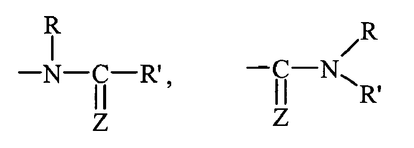 or a group 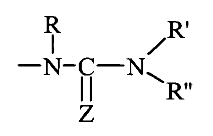 wherein :
• Z represents a sulphur atom or an oxygen atom,
• R and R", which may be identical or different, each represents a hydrogen atom or a linear or branched (C₁-C₆)alkyl group,
• and R' represents a linear or branched (C₁-C₆)alkyl group, a linear or branched (C₂-C₆)alkenyl group, a linear or branched (C₂-C₆)alkynyl group, a (C₃-C₈)-cycloalkyl group, a (C₃-C₈)cycloalkyl(C₁-C₆)alkyl group in which the alkyl moiety is linear or branched, an aryl group, an aryl-(C₁-C₆)alkyl group in which the alkyl moiety is linear or branched, a heteroaryl group or a heteroaryl-(C₁-C₆)alkyl group in which the alkyl moiety is linear or branched,
◆ X represents a nitrogen atom or a group N-R¹ wherein R¹ represents a hydrogen atom or a linear or branched (C₁-C₆)alkyl group, a (C₃-C₈)cycloalkyl group, a (C₃-C₈)cycloalkyl-(C₁-C₆)alkyl group in which the alkyl moiety is linear or branched, an aryl group, an aroyl group, an aryl-(C₁-C₆)alkyl group in which the alkyl moiety is linear or branched, a heteroaryl group, a heteroaroyl group or a heteroaryl-(C₁-C₆)alkyl group in which the alkyl moiety is linear or branched,
◆ R² represents a linear or branched (C₁-C₆)alkoxy group, a (C₃-C₈)cycloalkyloxy or (C₃-C₈)cycloalkyl-(C₁-C₆)alkyloxy group in which the alkyloxy moiety is linear or branched,
◆ the representation ----- denotes that the bond is single or double, with the proviso that the valency of the atoms is respected,
wherein :
- "aryl" is to be understood as meaning a phenyl or naphthyl group, those groups being unsubstituted or substituted by from one to three identical or different groups selected from linear or branched (C₁-C₆)alkyl, linear or branched (C₁-C₆)alkoxy, OH, COOH, alkoxycarbonyl in which the alkoxy moiety is linear or branched, formyl, nitro, cyano, hydroxymethyl, amino (optionally substituted by one or two linear or branched (C₁-C₆)alkyl groups) and halogen atoms,
- "heteroaryl" is to be understood as meaning any mono- or bi-cyclic group that contains from 5 to 10 ring members and may contain from 1 to 3 hetero atoms selected from oxygen, sulphur and nitrogen, such as the groups furan, thiophene, pyrrole, imidazoline, pyridine, quinoline, isoquinoline, chroman, indole, benzothiophene or benzofuran, it being possible for those groups to be partially hydrogenated, unsubstituted or substituted by from one to three identical or different groups selected from linear or branched (C₁-C₆)alkyl, linear or branched (C₁-C₆)alkoxy, OH, COOH, alkoxycarbonyl in which the alkoxy moiety is linear or branched, formyl, nitro, cyano, amino (optionally substituted by one or two linear or branched (C₁-C₆)alkyl groups), hydroxymethyl and halogen atoms,
their enantiomers and diastereoisomers, and also addition salts thereof with a pharmaceutically acceptable acid or base.

2. Compounds of formula (I) according to claim 1 wherein n is 2 and A represents an -NHCOR' group, their enantiomers and diastereoisomers, and also addition salts thereof with a pharmaceutically acceptable acid or base.

3. Compounds of formula (I) according to claim 1 wherein n is 3 and A represents an -CONHR' group, their enantiomers and diastereoisomers, and also addition salts thereof with a pharmaceutically acceptable acid or base.

4. Compounds of formula (I) according to claim 1 wherein R² represents a methoxy group, their enantiomers and diastereoisomers, and also addition salts thereof with a pharmaceutically acceptable acid or base.

5. Compounds of formula (I) according to claim 1 wherein X represents a nitrogen atom, their enantiomers and diastereoisomers, and also addition salts thereof with a pharmaceutically acceptable acid or base.

6. Compounds of formula (I) according to claim 1 wherein X represents an NPh or NBz group, their enantiomers and diastereoisomers, and also addition salts thereof with a pharmaceutically acceptable acid or base.

7. Compound of formula (I) according to claim 1 which is N-[2-(6-methoxy-4-isoquinolinyl)ethyl]acetamide, and also its addition salts with a pharmaceutically acceptable acid.

8. Compound of formula (I) according to claim 1 which is N-[2-(6-methoxy-4-isoquinolinyl)ethyl]butanamide, and also its addition salts with a pharmaceutically acceptable acid.

9. Compound of formula (I) according to claim 1 which is N-[2-(6-methoxy-4-isoquinolinyl)ethyl]propanamide, and also its addition salts with a pharmaceutically acceptable acid.

10. Compound of formula (I) according to claim 1 which is N-[2-(6-methoxy-4-isoquinolinyl)ethyl]cyclopropanecarboxamide, and also its addition salts with a pharmaceutically acceptable acid.

11. Compound of formula (I) according to claim 1 which is 4-(6-methoxy-4-isoquinolinyl)-*N*-methylbutanamide, and also its addition salts with a pharmaceutically acceptable acid.

12. Compound of formula (I) according to claim 1 which is N-[2-(6-methoxy-2-phenyl-1,2,3,4-tetrahydro-4-isoquinolinyl)ethyl]acetamide, and also its addition salts with a pharmaceutically acceptable acid.

13. Compound of formula (I) according to claim 1 which is N-[2-(2-benzyl-6-methoxy-1,2,3,4-tetrahydro-4-isoquinolinyl)ethyl]acetamide, and also its addition salts with a pharmaceutically acceptable acid.

14. Compound of formula (I) according to claim 1 which is N-{2-[2-(cyclopropyhnethyl-6-methoxy-1,2,3,4-tetrahydro-4-isoquinolinyl]ethyl}acetamide, and also its addition salts with a pharmaceutically acceptable acid.

15. Process for the preparation of compounds of formula (I) according to claim 1, **characterised in that** there is used as starting material a compound of formula (II) : 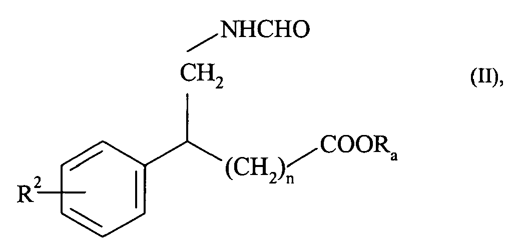 wherein R² and n are as defined for formula (I) and Rₐ represents a linear or branched (C₁-C₆)alkyl group, which is subjected to the action of POCl₃ to obtain a compound of formula (III) : 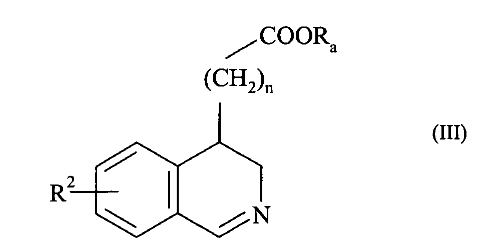 wherein R², n and Rₐ are as defined hereinabove, which is placed :
- in the presence of palladium-on-carbon to obtain a compound of formula (IV) : 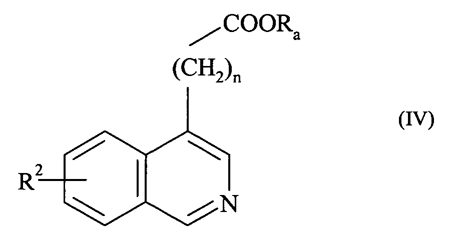 wherein R², n and Rₐ are as defined hereinabove,
- or which is hydrogenated in the presence of palladium-on-carbon to yield a compound of formula (V) : 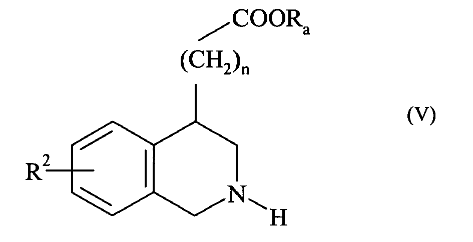
wherein R², n and Rₐ are as defined hereinabove,
which compound of formula (V) is condensed with a compound of formula G-R'¹ wherein G represents a leaving group and R'¹ may have any of the meanings given for R¹ with the exception of a hydrogen atom, to yield a compound of formula (VI) : 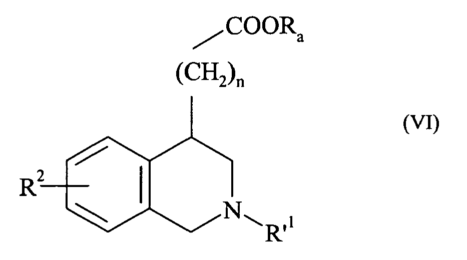 wherein R², R'¹, n and Rₐ are as defined hereinabove, the compounds of formulae (III) to (VI) constituting the compound of formula (VII) : 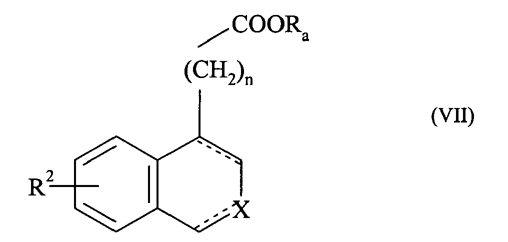 wherein R², n and Rₐ are as defined hereinabove and X and the representation ----- are as defined for formula (I),
which compound of formula (VII) is condensed with an amine of formula HNRR' wherein R and R' are as defined for formula (I) to obtain a compound of formula (I/a), a particular case of the compounds of formula (I) : 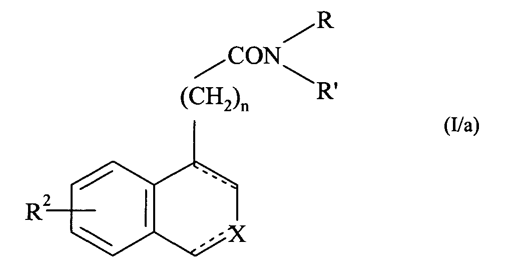 wherein R², R, R', X, n and the representation ----- are as defined hereinabove, or which compound of formula (VII) is subjected to a sequence of reactions conventional in organic chemistry to yield a compound of formula (VIII) : 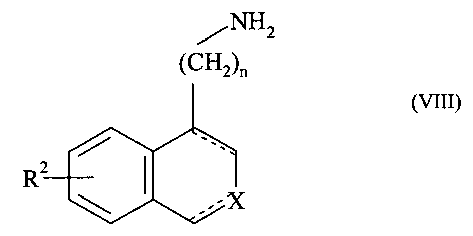 wherein R², X, n and the representation ----- are as defined hereinabove, which is :
- either reacted with an acyl chloride C1COR' or the corresponding mixed or symmetric anhydride wherein R' is as defined hereinabove to yield a compound of formula (I/b), a particular case of the compound of formula (I) : 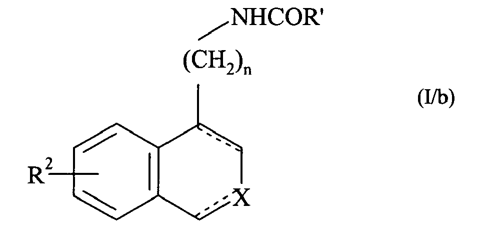 wherein R², R', X, n and the representation ----- are as defined hereinabove, optionally followed by the action of a compound of formula R'ₐ-J wherein R'ₐ may have any of the meanings of R' and J represents a leaving group, such as a halogen atom or a tosyl group, to obtain a compound of formula (I/c), a particular case of the compounds of formula (I) : 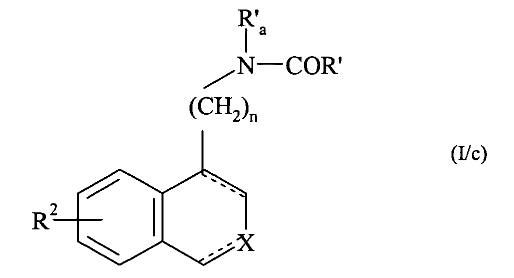 wherein R², R', Rₐ', X, n and the representation ----- are as defined hereinabove,
- or subjected to the action of a compound of formula (IX) :
O=C=N-R' (IX)
wherein R' is as defined hereinabove, optionally followed by the action of a compound of formula R'ₐ-J as defined hereinabove, to yield a compound of formula (I/d), a particular case of the compounds of formula (I) : 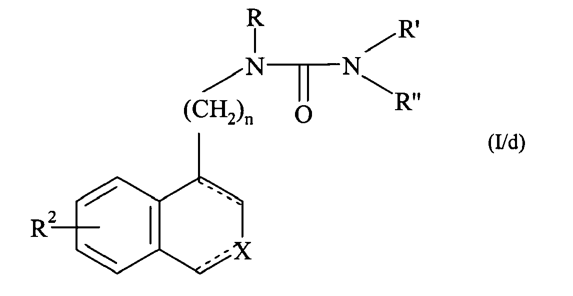 wherein R², R, R', n, X and the representation ----- are as defined hereinabove and R" is as defined for formula (I), it being possible for the compounds of formulae (I/a) to (I/d) to be subjected to the action of a thionisation agent, such as Lawesson's reagent for example, to yield a compound of formula (I/e), a particular case of the compounds of formula (I) : 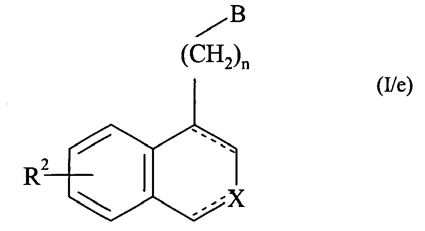 wherein R², n and the representation ----- are as defined hereinabove and B represents a C(S)NRR', N(R)C(S)R' or N(R)C(S)NR'R" group wherein R, R' and R" are as defined hereinabove,
the compounds (I/a) to (I/e) constituting the totality of the compounds of formula (I), which compounds may be purified according to a conventional separation technique, are converted, if desired, into addition salts with a pharmaceutically acceptable acid or base, and are optionally separated into the isomers according to a conventional separation technique.

16. Pharmaceutical compositions comprising the compounds of formula (I) according to any one of claims 1 to 14 or an addition salt thereof with a pharmaceutically acceptable acid or base in combination with one or more pharmaceutically acceptable excipients.

17. Pharmaceutical compositions according to claim 16 for use in the manufacture of medicaments for treating disorders of the melatoninergic system.

18. Pharmaceutical compositions according to claim 16 for use in the manufacture of medicaments for the treatment of sleep disorders, stress, anxiety, seasonal affective disorders or major depression, cardiovascular pathologies, pathologies of the digestive system, insomnia and fatigue due to jetlag, schizophrenia, panic attacks, melancholia, appetite disorders, obesity, insomnia, psychotic disorders, epilepsy, diabetes, Parkinson's disease, senile dementia, various disorders associated with normal or pathological ageing, migraine, memory loss, Alzheimer's disease, cerebral circulation disorders, and also in sexual dysfunction, as inhibitors of ovulation, immunomodulators, and in the treatment of cancers.

## Patentansprüche

1. Verbindungen der Formel (I): 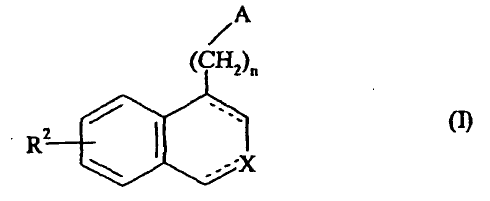 in der:
◆ n 1, 2 oder 3 bedeutet,
◆ A eine Gruppe 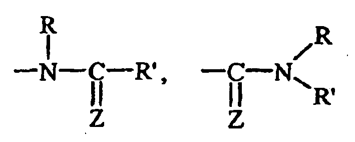 oder eine Gruppe 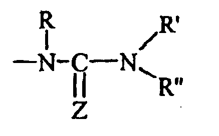 bedeutet, in denen:
• Z ein Schwefel- oder Sauerstoffatom darstellt,
• R und R", die gleichartig oder verschieden sind, jeweils ein Wasserstoffatom oder eine geradkettige oder verzweigte (C₁-C₆)-Alkylgruppe darstellen.
• und R' eine geradkettige oder verzweigte (C₁-C₆)-Alkylgruppe, geradkettige oder verzweigte (C₂-C₆)-Alkenylgruppe, geradkettige oder verzweigte (C₂-C₆)-Alkinylgruppe, (C₃-C₈)-Cycloalkylgruppe, (C₃-C₈)-Cycloalkyl-(C₁-C₆)-alkylgruppe, deren Alkylrest geradkettig oder verzweigt ist. Arylgruppe, Aryl-(C₁-C₆)-alkylgruppe, deren Alkylrest geradkettig oder verzweigt ist. Heteroarylgruppe oder Heteroaryl-(C₁-C₆)-alkylgruppe, deren Alkylrest geradkettig oder verzweigt ist, darstellt,
◆ X ein Stickstoffatom oder eine Gruppe N-R' bedeutet, worin R' ein Wasserstoffatom oder eine geradkettige oder verzweigte (C₁-C₆)-Alkylgruppe, (C₃-C₈)-Cycloalkylgruppe, geradkettige oder verzweigte (C₃-C₈)-Cyclo-(C₁-C₆)-alkylgruppe, Arylgruppe, Aroylgruppe oder Aryl-(C₁-C₆)-alkylgruppe, deren Alkylrest geradkettig oder verzweigt ist, Heteroarylgruppe, Heteroaroylgruppe oder Heteroaryl-(C₁-C₆)-alkylgruppe, deren Alkylrest geradkettig oder verzweigt ist, bedeutet,
◆ R² eine geradkettige oder verzweigte (C₁-C₆)-Alkoxygruppe, (C₃-C₈)-Cycloalkyloxygruppe oder geradkettige oder verzweigte (C₃-C₈)-Cycloalkyl-C₁-C₆)-alkyloxygruppe bedeutet und
◆ das Symbol ----- bedeutet, daß die Bindung einfach oder doppelt ist, mit der Maßgabe, daß die Wertigkeit der Atome berücksichtigt ist, mit der Maßgabe, daß:
- man unter "Aryl" eine Phenyl- oder Naphthylgruppe versteht, wobei diese Gruppen unsubstituiert oder durch eine bis drei gleichartige oder verschiedenartige Gruppen substituiert sind, ausgewählt aus geradkettigem oder verzweigtem (C,-C₆)-Alkyl, geradkettigem oder verzweigtem (C₁-C₆)-Alkoxy, OH, COOH, Alkoxycarbonyl, dessen Alkoxyrest geradkettige oder verzweigt ist, Formyl, Nitro, Cyano, Hydroxymethyl, Amino (gegebenenfalls substituiert durch eine oder zwei geradkettige oder verzweigte (C₁-C₆)-Alkylgruppen) oder Halogenatome,
- man unter "Heteroaryl" jede mono- oder bicyclische Gruppe versteht, die 5 bis 10 Kettenglieder aufweist und 1 bis 3 Heteroatome ausgewählt aus Sauerstoff, Schwefel oder Stickstoff enthält, wie Furan-, Thiophen-, Pyrrol-, Imidazolin-, Pyridin-, Chinolin-, Isochinolin-, Chroman-, Indol-, Benzothiophen- oder Benzofuran-Gruppen, wobei diese Gruppen teilweise hydriert, nichtsubstituiert oder durch eine bis drei gleichartige oder verschiedenartige Gruppen substituiert sein können, ausgewählt aus geradkettigem oder verzweigtem (C₁-C₆)-Alkyl, geradkettigem oder verzweigtem (C₁-C₆)-Alkoxy, OH, COOH, Alkoxycarbonyl, dessen Alkoxyrest geradkettig oder verzweigt ist, Formyl, Nitro, Cyano, Amino (gegebenenfalls substituiert durch eine oder zwei geradkettige oder verzweigte (C₁-C₆)-Alkylgruppen), Hydroxymethyl oder Halogenatomen,
deren Enantiomere und Diastereoisomere sowie deren Additionssalze mit einer pharmazeutisch annehmbaren Säure oder Base.

2. Verbindungen der Formel (I) nach Anspruch 1, worin n 2 bedeutet und A eine Gruppe -NHCOR' darstellt, deren Enantiomere und Diastereoisomere sowie deren Additionssalze mit einer pharmazeutisch annehmbaren Säure oder Base.

3. Verbindungen der Formel (1) nach Anspruch 1, worin n 3 bedeutet und A eine Gruppe -CONHR' darstellt, deren Enantiomere und Diastereoisomere sowie deren Additionssalze mit einer pharmazeutisch annehmbaren Säure oder Base.

4. Verbindungen der Formel (I) nach Anspruch 1, worin R² eine Methoxygruppe bedeutet, deren Enantiomere und Diastereoisomere sowie deren Additionssalze mit einer pharmazeutisch annehmbaren Säure oder Base.

5. Verbindungen der Formel (I) nach Anspruch 1, worin X ein Stickstoffatom bedeutet, deren Enantiomere und Diastereoisomere sowie deren Additionssalze mit einer pharmazeutisch annehmbaren Säure oder Base.

6. Verbindungen der Formel (I) nach Anspruch 1, worin X eine Gruppe NPh oder NBz bedeutet, deren Enantiomere und Diastereoisomere sowie deren Additionssalze mit einer pharmazeutisch annehmbaren Säure oder Base.

7. Verbindung der Formel (I) nach Anspruch 1, nämlich N-[2-(6-Methoxy-4-isochinolinyl)-ethyl]-acetamid sowie deren Additionssalze mit einer pharmazeutisch annehmbaren Säure.

8. Verbindung der Formel (I) nach Anspruch 1, nämlich N-[2-(6-Methoxy-4-isochinolinyl)-ethyl]-butanamid sowie deren Additionssalze mit einer pharmazeutisch annehmbaren Säure.

9. Verbindung der Formel (I) nach Anspruch 1, nämlich N-[2-(6-Methoxy-4-isochinolinyl)-ethyl]-propanamid sowie deren Additionssalze mit einer pharmazeutisch annehmbaren Säure.

10. Verbindung der Formel (I) nach Anspruch 1, nämlich N-[2-(6-Methoxy-4-isochinolinyl)-ethyl]-cyclopropancarboxamid sowie deren Additionssalze mit einer pharmazeutisch annehmbaren Säure.

11. Verbindung der Formel (I) nach Anspruch 1, nämlich 4-(6-Methoxy-4-isochinolinyl)-N-methylbutanamid sowie deren Additionssalze mit einer pharmazeutisch annehmbaren Säure.

12. Verbindung der Formel (I) nach Anspruch 1, nämlich N-12-(6-Methoxy-2-phenyl-1,2,3,4-tetrahydro-4-isochinolinyl)-ethyl]-acetamid sowie deren Additionssalze mit einer pharmazeutisch annehmbaren Säure.

13. Verbindung der Formel (I) nach Anspruch 1, nämlich N-[2-(2-Benzyl-6-methoxy-1,2,3,4-tetrahydro-4-isochinolinyl)-ethyl]-acetamid sowie deren Additionssalze mit einer pharmazeutisch annehmbaren Säure.

14. Verbindung der Formel (I) nach Anspruch 1, nämlich N-{2-[2-(Cyclopropylmethyl)-6-methoxy-1,2,3,4-tetrahydro-4-isochinolinyl]-ethyl}-acetamid sowie deren Additionssalze mit einer pharmazeutisch annehmbaren Säure.

15. Verfahren zur Herstellung der Verbindungen der Formel (I) nach Anspruch 1, **dadurch gekennzeichnet, daß** man als Ausgangsprodukt die Verbindung der Formel (II) verwendet: 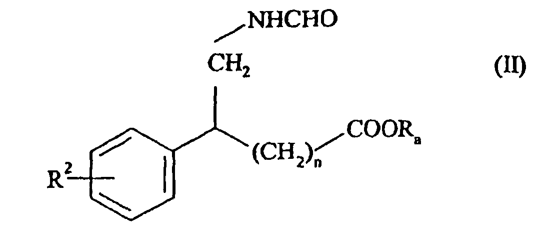 in der R² und n die bezüglich der Formel (I) angegebenen Bedeutungen besitzen und Rₐ eine geradkettige oder verzweigte (C₁-C₆)-Alkylgruppe darstellt, welche man der Einwirkung von POCl₃ unterwirft zur Bildung der Verbindung der Formel (III): 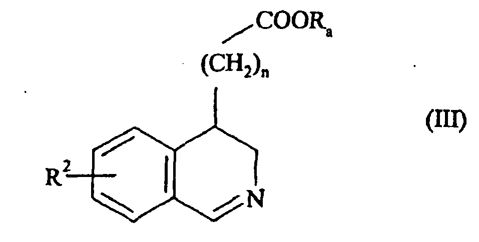 in der R², n und Rₐ die oben angegebenen Bedeutungen besitzen, welche man:
- in Gegenwart von Palladium-auf-Kohlenstoff zur Bildung der Verbindung der Formel (IV): 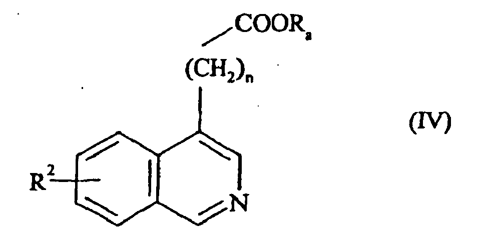 in der R², n und Rₐ die oben angegebenen Bedeutungen besitzen,
- oder in Gegenwart von Palladium-auf-Kohlenstoff hydriert zur Bildung der Verbindung der Formel (V): 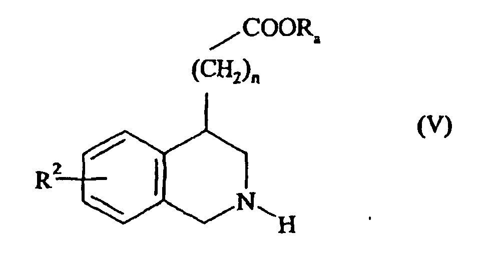 in der R², n und Rₐ die oben angegebenen Bedeutungen besitzen, welche Verbindung der Formel (V) man mit einer Verbindung der Formel G-R'¹ kondensiert, in der G eine austretende Gruppe bedeutet und R'¹ sämtliche Bedeutungen von R¹ annehmen kann mit Ausnahme des Wasserstoffatoms, zur Bildung der Verbindung der Formel (VI): 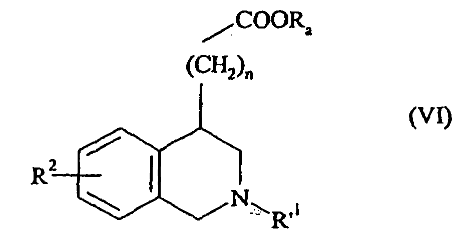 in der R², R'¹, n und Rₐ die oben angegebenen Bedeutungen besitzen,
welche Verbindungen der Formeln (III) bis (VI) die Verbindungen der Formel (VII) bilden: 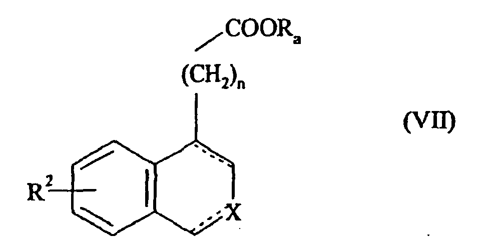 in der R², n und Rₐ die oben angegebenen Bedeutungen besitzen und X und das Symbol ----- die bezüglich der Formel (I) angegebenen Bedeutungen besitzen,
welche man mit einem Amin der Formel HNRR' kondensiert, in der R und R' die bezüglich der Formel (I) angegebenen Bedeutungen besitzen, zur Bildung einer Verbindung der Formel (I/a), einem Sonderfall der Verbindungen der Formel (I): 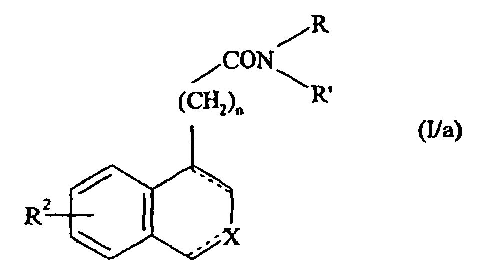 in der R², R, R', X, n und das Symbol ----- die oben angegebenen Bedeutungen besitzen,
oder welche Verbindung der Formel (VII) man der Einwirkung einer klassischen Reaktionsfolge der organischen Chemie unterwirft zur Bildung der Verbindung der Formel (VIII): 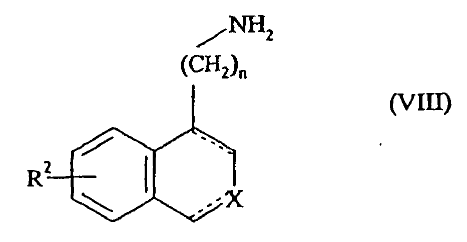 in der R², X, n und das Symbol ----- die oben angegebenen Bedeutungen besitzen, welche man:
- entweder mit einem Acylchlorid Cl COR' oder dem entsprechenden gemischten oder symmetrischen Anhydrid, worin R' die oben angegebenen Bedeutungen besitzt, umsetzt zur Bildung der Verbindung der Formel (I/b), einem Sonderfall der Verbindungen der Formel (I): 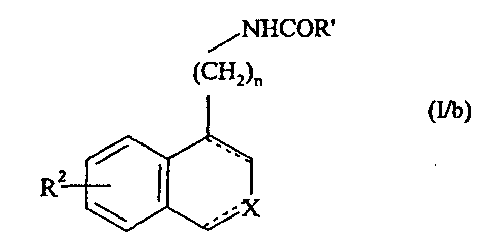 in der R², R', X, n und das Symbol ----- die oben angegebenen Bedeutungen besitzen, gegebenenfalls gefolgt von der Einwirkung einer Verbindung der Formel R'ₐ-J, worin R'ₐ sämtliche Bedeutungen von R' besitzen kann und J eine austretende Gruppe, wie ein Halogenatom oder eine Tosylgruppe darstellt, zur Bildung der Verbindung der Formel (I/c), einem Sonderfall der Verbindungen der Formel (I): 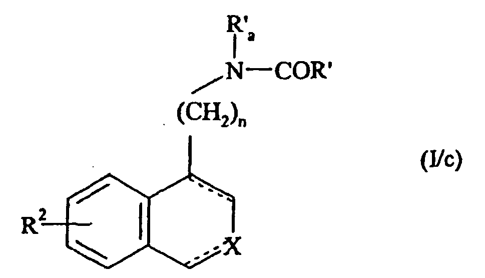 in der R², R', Rₐ', X, n und das Symbol ----- die oben angegebenen Bedeutungen besitzen,
- oder der Einwirkung einer Verbindung der Formel (IX) unterwirft:
O = C = N-R' (IX)
in der R' die oben angegebenen Bedeutungen besitzt, gegebenenfalls gefolgt von der Einwirkung einer Verbindung der Formel R'ₐ-J, wie sie oben definiert worden ist, zur Bildung der Verbindung der Formel (I/d), einem Sonderfall der Verbindungen der Formel (I): 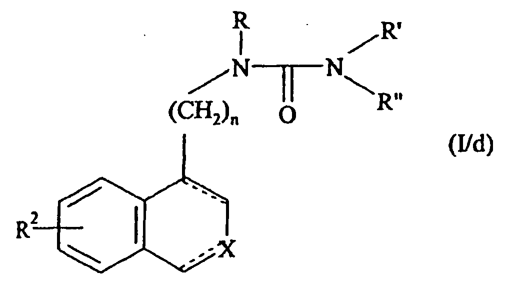 in der R², R, R', n, X und das Symbol ----- die oben angegebenen Bedeutungen besitzen und R" die bezüglich der Formel (I) angegebenen Bedeutungen aufweist,
welche Verbindungen der Formeln (I/a) bis (I/d) der Einwirkung eines Thionierungsmittels, wie beispielsweise des Lawesson-Reagens, unterworfen werden können zur Bildung der Verbindung der Formel (I/e), einem Sonderfall der Verbindungen der Formel (I): 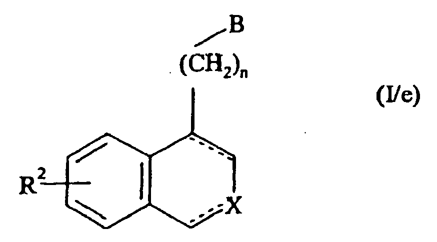 in der R², n und das Symbol ----- die oben angegebenen Bedeutungen besitzen und B eine Gruppe C(S)NRR', N(R)C(S)R' oder N(R)C(S)NR'R" bedeutet, worin R, R' und R" die oben angegebenen Bedeutungen aufweisen,
wobei die Verbindungen (I/a) bis (I/e) die Gesamtheit der Verbindungen der Formel (I) bilden und mit Hilfe einer klassischen Trennmethode gereinigt werden können, gewünschtenfalls in ihre Additionssalze mit einer pharmazeutisch annehmbaren Säure oder Base überführt werden können und gegebenenfalls mit Hilfe einer klassischen Trennmethode in ihre Isomeren aufgetrennt werden können.

16. Pharmazeutische Zubereitungen enthaltend die Verbindungen der Formel (I) nach einem der Ansprüche 1 bis 14 oder eines ihrer Additionssalze mit einer pharmazeutisch annehmbaren Säure oder Base in Kombination mit einem oder mehreren pharmazeutisch annehmbaren Hilfsstoffen.

17. Pharmazeutische Zubereitungen nach Anspruch 16, nützlich für die Herstellung von Arzneimitteln zur Behandlung von Störungen des melatoninergischen Systems.

18. Pharmazeutische Zubereitungen nach Anspruch 16, nützlich für die Herstellung von Arzneimitteln zur Behandlung von Schlafstörungen, Streß, Angst, saisonal bedingten Depressionen oder schweren Depressionen, kardiovaskulären Erkrankungen, Erkrankungen des Verdauungssystems, Schlaflosigkeit und Müdigkeit als Folge von Zeitverschiebungen, der Schizophrenie, von Panikanfällen, der Melancholie, von Appetitstörungen, der Fettsucht, der Schlaflosigkeit, von psychischen Störungen, der Epilepsie, des Diabetes, der Parkinsonschen Krankheit, der senilen Demenz, von verschiedenen Störungen, die mit dem normalen oder pathologischen Altern verknüpft sind, von Migräne, Gedächtnisverlusten, der Alzheimerschen Krankheit, Störungen der Gehirndurchblutung sowie sexuellen Dysfunktionen, als Inhibitoren der Ovulation, Immunomodulatoren und bei der Behandlung von Krebs.
